# EUROPEAN PATENT APPLICATION

(11) **EP 3 587 416 A1**
(43) Date of publication of application: **01.01.2020**
(21) Application number: 18382487.9
(22) Date of filing: 29.06.2018
(51) Int. Cl.: C07D 409/12, C07D 409/14, C07D 401/12, C07D 401/14, C07D 413/12, C07D 413/14, C07D 211/76

(54) **2-OXOPIPERIDIN-3-YL DERIVATIVES AND USE THEREOF**

(71) Applicant: Institut Univ. de Ciència i Tecnologia, S.A., 08100 Mollet del Vallès Barcelona (ES)
(72) Inventor: PASCUAL GILABERT, Marta, 08100 MOLLET DEL VALLÈS (ES); PÉREZ OZCÁRIZ, Sergio, 08100 MOLLET DEL VALLES (ES); LÓPEZ GÓMEZ, Cristina, 08100 MOLLET DEL VALLÈS (ES); ALONSO FERNÁNDEZ, Javier, 08100 MOLLET DEL VALLÈS (ES); CASTELLS BOLIART, Josep, 08100 MOLLET DEL VALLÈS (ES)
(74) Representative: Ponti & Partners, S.L.P

(57) **Abstract**

Tthe present invention relates to a novel compound of formula (I). The present invention also relates to the use of a compound of formula (I) as a calcineurin inhibitor. The present invention further relates to a compound of formula (I) for use in the prevention or treatment of an autoimmune disease or organ transplant rejection.

## Description

### Field of the invention

The present invention relates to novel compounds of formula (I) and to their use as calcineurin inhibitors in the prevention or treatment of an autoimmune disease or organ transplant rejection.

### Background of the invention

The immune system is a complex network of cells and molecules that work together to protect the body against various diseases. Hyperactivity or inappropriate activity of the immune system is a serious and widespread medical problem. It contributes to acute and chronic immune diseases, e.g., allergic and atopic diseases, asthma, allergic rhinitis, allergic conjunctivitis and atopic dermatitis, and in certain circumstances, the immune system may also attack and damage the body's own tissues, organs and cells, resulting in autoimmune diseases (ADs).

Almost any part of the body can be targeted by the immune system, including the heart, brain, nerves, muscles, skin, eyes, lungs, the digestive tract and blood vessels. A broad range of ADs exist given that they vary according to the part of the body that is being targeted by the immune system. Currently, more than 100 different types of ADs have been identified. Common ADs include rheumatoid arthritis, systemic lupus erythematosus, multiple sclerosis, type 1 diabetes, psoriasis, and celiac diseases (for a complete list of said ADs, see WO 2017/212018 (pages 3-7), incorporated herein by reference). In fact, ADs affect 5 to 10% of the global population, particularly women, who are two to ten times more likely to suffer from an AD than men. Some of the ADs are within the top 10 leading causes of death among women aged 65 and older (Autoimmune Diseases - Modern Diseases, Study for the ENVI Committee, European Parliament, 2017, and references cited therein, available at http://www.europarl.europa.eu/cmsdata/133620/ENVI%202017-09%20WS%20Autoimmune%20diseases%20%20PE%20614.174%20(Publication).pdf).

Hyperactivity or inappropriate activity of the immune system is also involved in transplant graft rejections and graft-versus-host disease. Rejection takes place when the organ recipient's immune system recognizes the donor organ as foreign and attempts to eliminate it. Common organs transplanted include, but are not limited to, kidney, liver, heart, lung, and pancreas, among others. Accordingly, immunosupressive drugs prevent the graft vs host reactions or tissue rejections after allo- or xenotransplantation.

A certain family of transcription factors, the NFAT proteins (also named NFATc), are expressed in immune cells and play a key role in eliciting immune responses. The NFAT proteins are activated by calcineurin, and the activated NFAT proteins, in turn, induce transcription of cytokine genes which are required for an immune response. The immunosuppressive drugs cyclosporin A and tacrolimus (FK506) are potent inhibitors of cytokine gene transcription in activated immune cells, and have been reported to act by inhibiting calcineurin such that calcineurin is not able to activate NFAT. These drugs, however, can display nephrotoxic and neurotoxic effects after long term usage. Since calcineurin is ubiquitously expressed in many tissues, the drugs' inhibition of calcineurin activity toward substrates other than NFAT may contribute to the observed toxicity.

Calcineurin (CN or PPP3, formerly PP2B) is a serine/threonine protein phosphatase regulated by Ca2+ and calmodulin. CN regulates development, memory, cardiac function, and immune response by dephosphorylating a variety of protein substrates, including the family of cytosolic Nuclear Factor of Activated T-cells (NFATc) transcription factors. NFATc proteins have been shown to be direct substrates of calcineurin. Dephosphorylated NFATc proteins translocate into the nucleus where, in cooperation with other transcription factors, induce the expression of many cytokine and chemokine genes, leading to T cell activation.

Calcineurin plays a prominent role in many physiological processes, including homeostasis, angiogenesis, myogenesis, adipogenesis, osteogenesis, chondrocyte differentiation, cardiovascular system development, pancreatic β-cell proliferation, hair follicle cell differentiation and remodelling, and activities of cells of the immune and nervous systems, including schizophrenia, diabetes mellitus, Down synthdrome, cardiac hypertrophy, as well as in neurodegenerative diseases, such as Alzheimer's disease, psychotic disorders, epilepsy, among others (Erdmann, F et al. Calcineurin inhibitors: status quo and perspectives. BioMol Concepts, 2011, vol. 2, pp. 65-78; Kipanyula, M.J. et al The emerging roles of the Calcineurin-Nuclear Factor of activated T-Lymphocyte pathway in nervous system functions and diseases, J. Aging Research, 2016, Article ID 5081021, http://dx.doi.org/10.1155/2016/5081021).

The immunosuppressant drugs cyclosporin A (CsA) and tacrolimus (FK506) are the cornerstone of immunosuppressive therapy. These drugs, in complex with endogenous immunophilins, sterically hinder the phosphatase activity of CN towards NFATc and all other substrates, by blocking the LxVP binding site of CN. Both drugs have narrow therapeutic windows. As a consequence, despite their benefits, severe side effects, such as neurotoxicity, renal dysfunction, hypertension and diabetes, have been related to long-term administration (Review: Azzi, J. R. et al. Calcineurin Inhibitors: 40 Years Later, Can't Live Without... J. Immunol. December 15, 2013, 191 (12) 5785-5791; DOI: https://doi.org/10.4049/jimmunol.1390055).

Siebert, M et al (Novel inhibitors of the calcineurin/NFATc hub-aternatives to CsA and FK506? Cell Communication and Signaling, 2009, 7:25 doi:10.1189/1478-811X-7-25) and Erdmann, F *et al* (vide supra) review novel inhibitors of the calcineurin/NFATc signalling pathway. Moreover, they disclose that compounds that are able to discriminate not only between the inhibition of calcineurin and PPlases (peptidiyl-prolyl *cis-trans* isomerase) as well as of NFATc and other substrates of calcineurin might cause fewer side effects in clinical applications. Not a single one of the substances disclosed therein have been introduced in clinical trials so far.

According to the prior art, the CN docking site of NFATc is mainly formed by the PxIxIT motif, the main anchoring site, and the LxVP motif, to properly position the substrate phosphoresidue towards the catalytic active site (Li, H. et al. Interaction of calcineurin with substrates and targeting proteins. Trends in Cell Biology, 2011, 21 (2), 91-103; Matsoukas, Minos et al (2015). Identification of small-molecule inhibitors of calcineurin-NFATc signaling that mimic the PxIxIT motif of calcineurin binding partners. Science Signaling. 8. 10.1126/scisignal.2005918 & Fig. S1 in supplementary information).

Perez-Riba et al (WO2016207212 A1) and Matsoukas *et al* (vide supra) disclose non-peptide inhibitors of the calcineurin -NFAT signalling pathway that disrupt the CNA-NFTAc interaction without blocking general calcineurin phosphatase activity through specifically binding of the compounds disclosed therein to the PxIxIT binding region of calcineurin.

WO 02/102380 A1 discloses monocyclic or bicyclic carbocycles and heterocycles which are useful as inhibitors of factor Xa for the treatment and prevention of thromboembolic disorders. These compounds (especially those disclosed in page 152, Table 1 and page 156, Table 2) are not considered to be part of the present invention.

US 2004/0006062 discloses sylfonylaminovalerolactams and derivatives thereof useful as inhibitors of trypsin-like serine proteases, specifically factor Xa. These compounds do not include the biphenilic ring system contained in the compounds of the formula (I) of the present invention. Accordingly, compounds in US 2004/0006062 are not the object of the present invention.

WO 02/00651 discloses inhibitors of trypsin-like serine protease enzymes, especially factor Xa as anticoagulant agents for treatment and prevention of thromboembolic disorders. Since ring M is a 5 membered aromatic heterocycle, these compounds are not the object of the present invention.

Therefore, it is necessary to develop alternative strategies for disrupting Ca2+-calcineurin-NFATc signalling pathway that overcome the problems associated to the methods based on the use of currently available immunosupressants to be more specific for the calcineurin/NFAT pathway. Additionally, there is a need for immunosuppressive agents which selectively inhibit the calcineurin-NFAT interactions and which do not inhibit the enzymatic activity of calcineurin for its other substrates.

Applicants of the present invention have surprisingly found that compounds of formula (I) compete with NFATc binding to CN by a different site from the previously described PxIxIT- and LxVP-binding motifs on CNA. The above mentioned pocket may be considered as a novel binding site for calcineurin inhibition.

### Summary of the invention

In a first aspect the present invention relates to a novel compound of formula (I).

In a second aspect, the present invention relates to the use of a compound of formula (I) as a calcineurin inhibitor.

In a third aspect, the present invention relates to a compound of formula (I) for use in the prevention or treatment of an autoimmune disease or organ transplant rejection.

### Brief description of the drawings

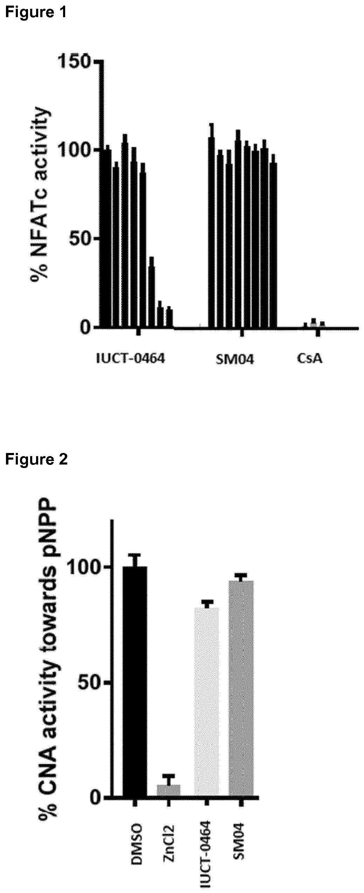

**Figure 1****. Effect of INK compounds on NFATc activation in HEK 293T cells.** Cells were transfected with 3xNFAT-luc reporter gene, treated with each compound at 0 µM / 0.02 µM / 0.2 µM / 1 µM / 5 µM/ 20 µM / 50 µM /100 µM (increasing concentrations of the compounds are represented in the graph from left to right) for 2 hours. Then, cells were calcium stimulated with ionomycin (1 µM), PMA (20 nM) and CaCl2 (10 mM) for 4 hours and luciferase activity was analyzed following the dual luciferase reporter assay manufacters' indications and a luminometer (Victor X5 plate reader form Perin-Elmer). NFATc activation upon cell stimulation, in the absence of any compound, was considered as 100 % (positive control of CN activation). Cells were treated with 1 µM of CsA 30 min prior Io/PMA/CaCl2 stimulation as negative control of NFATc activation. Values are given as percentage of NFATc activation relative to the activity of the positive control (100%) as mean ± SEM of 3 independent experiments with triplicates.
**Figure 2****: INK compounds do not affect CN phosphatase activity towards the pNPP substrate.** Results are shown as percentage of CNA phosphatase activity in the presence of 50 µM of each compound relative to the same activity in the absence of any compound. Data is given as mean percentage ± SD of 2 independent experiments with triplicates. As a negative control 50 µM ZnCl2 was used.

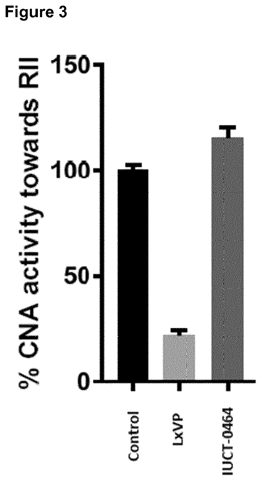

**Figure 3****: Compounds of formula (I) do inhibit phosphatase activity of CN towards the RII substrate.** CN activity towards the RII peptide was analyzed in the absence (positive control of CN activity) or in the presence of each compound at 50 µM, or of 50 µM of LxVP peptide (positive control of CN activity inhibition). Data is given as mean percentage ± SEM of 2 independent experiments with triplicates.

### Detailed description of the invention

The present invention relates to a compound having formula (I) 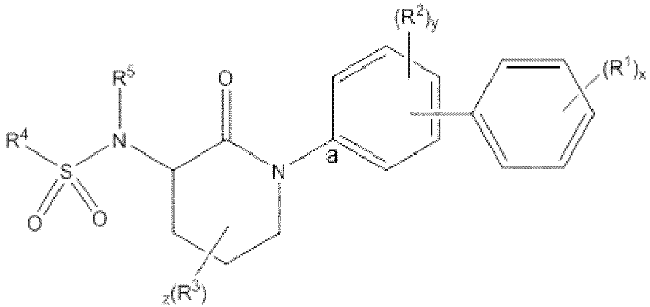 and the salts and stereoisomers thereof, wherein
**R¹** is hydrogen; OH; NH₂; SH; F; Cl; Br; I; methyl; an optionally substituted alkyl chain provided that said optionally substituted alkyl chain is not CH₂NEt₂, optionally substituted CH₂-pirrolidine, optionally substituted CH₂-piperidine or CH₂NMeCH₂CH₂OH; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; NO₂; N₃; SR⁸; SO₃H; SO₂R⁸; SO₂NR⁶R⁹; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
wherein **x** is one, two, three, four or five, independently of y, z;
wherein the phenyl ring bearing substituent **R¹** is in an orto, meta or para position regarding the position "a" of the phenyl ring bearing substituent **R²;**
**R²** is hydrogen; OH; NH₂; SH; F; Cl; Br; I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
wherein **y** is one, two, three, four or five, independently of x, z;
**R³** is hydrogen; OH; NH₂; SH; F; Cl; Br; I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
wherein **z** is one, two, three, four or five, independently of x, y;
**R⁴** is hydrogen; OH; NH₂; methyl; an optionally substituted alkyl chain; an optionally substituted alkenyl chain; an optionally substituted alkynyl chain; an optionally substituted cycloalkyl linked to S through an optionally substituted alkyl chain; an optionally substituted cycloalkyl linked to S through an optionally substituted alkenyl chain; an optionally substituted cycloalkyl linked to S through an optionally substituted alkynyl chain; an optionally substituted cycloalkenyl; an optionally substituted cycloalkenyl linked to S through an optionally substituted alkyl chain; an optionally substituted cycloalkenyl linked to S through an optionally substituted alkenyl chain; an optionally substituted cycloalkenyl linked to S through an optionally substituted alkynyl chain; an optionally substituted cycloalkynyl chain; an optionally substituted cycloalkynyl linked to S through an optionally substituted alkyl chain; an optionally substituted cycloalkynyl linked to S through an optionally substituted alkenyl chain; an optionally substituted cycloalkynyl linked to S through an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
**R⁵** is hydrogen; OH; NH₂; methyl; an optionally substituted alkyl chain; an optionally substituted alkenyl chain; an optionally substituted alkynyl chain; an optionally substituted cycloalkyl linked to S through an optionally substituted alkyl chain; an optionally substituted cycloalkyl linked to S through an optionally substituted alkenyl chain; an optionally substituted cycloalkyl linked to S through an optionally substituted alkynyl chain; an optionally substituted cycloalkenyl; an optionally substituted cycloalkenyl linked to S through an optionally substituted alkyl chain; an optionally substituted cycloalkenyl linked to S through an optionally substituted alkenyl chain; an optionally substituted cycloalkenyl linked to S through an optionally substituted alkynyl chain; an optionally substituted cycloalkynyl chain; an optionally substituted cycloalkynyl linked to S through an optionally substituted alkyl chain; an optionally substituted cycloalkynyl linked to S through an optionally substituted alkenyl chain; an optionally substituted cycloalkynyl linked to S through an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
**R⁶** and **R⁷** are selected, independently of each other, from hydrogen; methyl; an optionally substituted alkyl chain; an optionally substituted alkenyl chain; an optionally substituted alkynyl chain; an optionally substituted heterocycle; and an optionally substituted aryl, preferably phenyl or naphthyl;
**R⁸** is selected from hydrogen; an optionally substituted alkyl chain except methyl; an optionally substituted alkenyl chain; an optionally substituted alkynyl chain; an optionally substituted heterocycle; and an optionally substituted aryl, preferably phenyl or naphthyl;
**R⁹** is selected, independently of **R⁶,** from an optionally substituted alkyl chain; an optionally substituted alkenyl chain; an optionally substituted alkynyl chain; an optionally substituted heterocycle; and an optionally substituted aryl, preferably phenyl or naphthyl;
each **aryl** as a group or part of a group is phenyl, naphthalenyl, anthracenyl, phenantrenyl, biphenyl, optionally fused to Het; each optionally substituted with one, two, three or four substituents selected from OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain;
each **Het** as a group or part of a group is a monocyclic ring with 5, 6 or 7 ring atoms optionally fused to an aryl or a bicyclic ring structure comprising a 5, 6 or 7 membered ring linked to a 4, 5, or 6 membered ring; each of the rings being saturated, partially unsaturated, or completely unsaturated; at least one of the rings containing 1 to 5 heteroatoms each independently selected from nitrogen, oxygen and sulphur; and any one of the rings being optionally substituted with one, two, three or four substituents each independently selected from the group consisting of OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NO₂; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

In a preferred embodiment, the phenyl ring bearing substituent **R¹** is in para position regarding the position "a" of the phenyl ring bearing substituent **R².**

In another preferred embodiment, **R³** is hydrogen.

In a further preferred embodiment, **R²** is hydrogen; OH; NH₂; SH; F; Cl; Br; I; or methyl.

In a further preferred embodiment, **R⁴** is aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain.

In a further preferred embodiment, **R**¹ is hydrogen; F; Cl; Br; I; methyl; an optionally substituted alkyl chain; aryl; OR⁶ or Het;
provided that said optionally substituted alkyl chain is not CH₂NEt₂, optionally substituted CH₂-pirrolidine, optionally substituted CH₂-piperidine or CH₂NMeCH₂CH₂OH;
**R²** is F; Cl; Br; I;
**R⁵** is hydrogen; methyl; an optionally substituted alkyl chain; an optionally substituted cycloalkyl linked to S through an optionally substituted alkyl chain; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

In a further preferred embodiment, **R¹** is OR⁶.

In a further preferred embodiment, **R²** is F.

It should be noted that each of the previous embodiments can be considered independently from each other or each embodiment can be combined with one or more of the other embodiments.

In an even further preferred embodiment, the compound of formula (I) is selected from:
- 4,5-Dichloro-*N*-(1-(3-fluoro-2'-methoxy-[1,1'-biphenyl]-4-yl)-2-oxopiperidin-3-yl)thiophene-2-sulfonamide;
- *N*-(1-(3-fluoro-2'-methoxy-[1,1'-biphenyl]-4-yl)-2-oxopiperidin-3-yl)-3,5-dimethylisoxazole-4-sulfonamide;
- *N*-(1-(3-fluoro-2'-methoxy-[1,1'-biphenyl]-4-yl)-2-oxopiperidin-3-yl)benzo[d][1,3]dioxole-5-sulfonamide;
- *N*-(1-(3-fluoro-2'-methoxy-[1,1'-biphenyl]-4-yl)-2-oxopiperidin-3-yl)-5-(pyridin-2-yl)thiophene-2-sulfonamide;
- *N*-(1-(3-fluoro-2'-methoxy-[1,1'-biphenyl]-4-yl)-2-oxopiperidin-3-yl)benzofuran-2-sulfonamide;
- 4,5-Dichloro-*N*-(cyanomethyl)-*N*-(1-(3-fluoro-2'-methoxy-[1,1'-biphenyl]-4-yl)-2-oxopiperidin-3-yl)thiophene-2-sulfonamide;
- Ethyl *N*-(benzo[d][1,3]dioxol-5-ylsulfonyl)-*N*-(1-(3-fluoro-2'-methoxy-[1,1'-biphenyl]-4-yl)-2-oxopiperidin-3-yl)glycinate;
- *N*-(1-(3-fluoro-2'-methoxy-[1,1'-biphenyl]-4-yl)-2-oxopiperidin-3-yl)-*N*-(2-morpholino-2-oxoethyl)benzo[d][1,3]dioxole-5-sulfonamide;
- 2-((4,5-dichloro-*N*-(1-(3-fluoro-2'-methoxy-[1,1'-biphenyl]-4-yl)-2-oxopiperidin-3-yl)thiophene)-2-sulfonamido)acetamide;
- Ethyl *N*-(1-(3-fluoro-2'-methoxy-[1,1'-biphenyl]-4-yl)-2-oxopiperidin-3-yl)-*N*-((5-(pyridin-2-yl)thiophen-2-yl)sulfonyl)glycinate;
- *N*-(1-(3-fluoro-2'-methoxy-[1,1'-biphenyl]-4-yl)-2-oxopiperidin-3-yl)-5-(2-(methylthio) pyrimidin-4-yl)thiophene-2-sulfonamide;
- *N*-(1-(3-fluoro-2'-methoxy-[1,1'-biphenyl]-4-yl)-2-oxopiperidin-3-yl)benzo[b] thiophene-2-sulfonamide;
- *N*-(1-(3-fluoro-2'-methoxy-[1,1'-biphenyl]-4-yl)-2-oxopiperidin-3-yl)-5-(isoxazol-5-yl)thiophene-2-sulfonamide;
- *N*-(1-(3-fluoro-2'-methoxy-[1,1'-biphenyl]-4-yl)-2-oxopiperidin-3-yl)-5-(oxazol-5-yl)thiophene-2-sulfonamide;
- *N*-(1-(3-fluoro-2'-methoxy-[1,1'-biphenyl]-4-yl)-2-oxopiperidin-3-yl)-5-(2-methylthiazol-4-yl)thiophene-2-sulfonamide; and
- *N*-(1-(3-fluoro-2'-methoxy-[1,1'-biphenyl]-4-yl)-2-oxopiperidin-3-yl)-5-phenylthiophene-2-sulfonamide.

Preferably, the compound of formula (I) is selected from:
- *N*-(1-(3-fluoro-2'-methoxy-[1,1'-biphenyl]-4-yl)-2-oxopiperidin-3-yl)-5-(pyridin-2-yl)thiophene-2-sulfonamide; and
- *N*-(1-(3-fluoro-2'-methoxy-[1,1'-biphenyl]-4-yl)-2-oxopiperidin-3-yl)benzofuran-2-sulfonamide.

Unless specificially stated when defining the radicals (R) in the compound of formula (I), for the purposes of present description, the following terms are further defined as follows and applied as such.

The terms "heterocyclic ring" or "heterocycle" are used interchangeably herein and refer to any compound in which plurality of atoms form a ring via a plurality of covalent bonds, wherein the ring includes at least one atom other than a carbon atom. Particularly contemplated heterocyclic bases include 4-, 5- and 6-membered rings containing at least 1 to 4 heteroatoms each independently selected from nitrogen, oxygen and sulphur as the non-carbon atom (e.g., imidazole, pyrrole, triazole, dihydropyrimidine). Further contemplated heterocycles may be fused (i.e., covalently bound) to another ring or heterocycle, and are thus termed "fused heterocycle" or "fused heterocyclic base" as used herein. Especially contemplated fused heterocycles include a 5-membered ring fused to a 6-membered ring (e.g., purine, pyrrolo[2,3-*d*]pyrimidine), and a 6-membered ring fused to another 6-membered or higher ring (e.g., pyrido[4,5-*d*]pyrimidine, benzodiazepine). Still further contemplated heterocyclic bases may be aromatic, or may include one or more double or triple bonds. Moreover, contemplated heterocyclic bases and fused heterocycles may further be substituted in one or more positions. And any one of the rings being optionally substituted with one, two or three substituents each independently selected from the group consisting of halogen, hydroxy, nitro, cyano, carboxyl, C₁₋₆alkyl, C₁₋₆alkoxy, C₁₋₆alkoxyC₁₋₆alkyl, C₁₋₆alkylcarbonyl, amino, mono- or diC₁₋₆alkylamino, azido, mercapto, polyhaloC₁₋₆alkyl, polyhaloC₁₋₆alkoxy, and C₃₋₇cycloalkyl.

"Het" is defined herein as a group or part of a group being a monocyclic ring with 5, 6 or 7 ring atoms optionally fused to an aryl or a bicyclic ring structure comprising a 5, 6 or 7 membered ring linked to a 4, 5, or 6 membered ring; each of the rings being saturated, partially unsaturated, or completely unsaturated; at least one of the rings containing 1 to 5 heteroatoms each independently selected from nitrogen, oxygen and sulphur; and any one of the rings being optionally substituted with one, two, three or four substituents each independently selected from the group consisting of OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NO₂; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "tautomer" or "tautomeric form" refers to structural isomer of different energies which are interconvertible via a low energy barrier. For example, proton tautomers (also known as prototropic tautomers) include interconversion via migration of a proton, such as keto-enol and imine-enamine isomerizations. Valence tautomers include interconversions by reorganization of some of the bonding electrons.

The term "regioisomer" refers to structural isomer, or constitutional isomer in the sense that refers to molecules with the same molecular formula that whose atoms are bonded in different order of connectivity.

The term "conversion" refers to is the percentage of starting material that is transformed into products, either the expected final product, byproducts, or even into products of degradation.

The term "yield" is the number of synthesized molecules of product per number of starting molecules. In a multistep synthesis, the yield can be calculated by multiplication of the yields of all the single steps.

The term "anomeric purity" refers to the amount of a particular anomer of a compound divided by the total amount of all anomers of that compound present in the mixture multiplied by 100.

The term "intermediate" or "intermediates" refer to any compounds which may be transformed into the final product by means of suitable additional chemical reactions. The compounds of the present invention can also be considered as intermediate compounds and as such are also included in the scope of the present invention.

An *in vivo* hydrolysable ester of a compound of the formula (I) containing a hydroxy group includes inorganic esters such as phosphate esters and α-acyloxyalkyl ethers and related compounds which as a result of the *in vivo* hydrolysis of the ester breakdown to give the parent hydroxy group. Examples of α-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4-position of the benzoyl ring.

For therapeutic use, salts the compounds of formula (I) are those wherein the counter-ion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the scope of the present invention.

The pharmaceutically acceptable acid and base addition salts as mentioned above are meant to comprise the therapeutically active non-toxic acid and base addition salt forms which the compounds of formula (I) are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

Conversely said salt forms can be converted by treatment with an appropriate base into the free base form.

The compounds of formula (I) containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term "addition salt" as used hereinabove also comprises the solvates which either the compounds of formula (I) as well as the salts thereof, are able to form. Such solvates are for example hydrates, alcoholates and the like.

The term "quaternary amine" as used hereinbefore defines the quaternary ammonium salts which the compounds of formula (I) are able to form by reaction between a basic nitrogen of either the compounds of formula (I) or the compounds of formula (I)I and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

The N-oxide forms of the present compounds are meant to comprise the compounds of formula (I) wherein one or several nitrogen atoms are oxidized to the so-called N-oxide.

It will be appreciated that the compounds of formula (I) may have metal binding, chelating, complex forming properties and therefore may exist as metal complexes or metal chelates. Such metalated derivatives of the compounds of formula (I) are intended to be included within the scope of the present invention.

Some of the compounds of the compounds of formula (I) may also exist in their tautomeric form. Such forms although not explicitly indicated in the above formula are intended to be included within the scope of the present invention.

The compounds described herein may have asymmetric centers and occur as racemates, racemic mixtures, individual diastereomers or enantiomers, with all isomeric forms being included in the present invention. Compounds of the present invention having a chiral center can exist in and be isolated in optically active and racemic forms. Some compounds can exhibit polymorphism.

The term "alkyl" as used herein it does refer to any linear, branched, or cyclic hydrocarbon in which all carbon-carbon bonds are single bonds. Alkyl chains may optionally be substituted by OH; NH₂; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR₆; NR⁶R⁷; CN; COR₆; CONR₆R₇; CO₂R⁶; C(S)OR₆; OCONR₆R₇; OCOR₆; OCO₂R₆; OC(S)OR₆; N₃; NO₂; NHCONR₆R₇; NHCOR₆; NR₆CO₂R₇; NHCO₂R₆; NHC(S)OR₆; NO₂; SR₆; SO₃H; SO₂R₆; SO₂NR₆R₇; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "alkenyl" and "optionally substituted alkenyl" are used interchangeably herein and refer to any linear, branched, or cyclic alkyl with at least one carbon-carbon double bond. Alkenyl chains may optionally be substituted by OH; NH₂; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR₆; NR⁶R⁷; CN; COR₆; CONR₆R₇; CO₂R⁶; C(S)OR₆; OCONR₆R₇;OCOR₆; OCO₂R₆; OC(S)OR₆; N₃; NO₂; NHCONR₆R₇; NHCOR₆; NR₆CO₂R₇; NHCO₂R₆; NHC(S)OR₆; NO₂; SR₆; SO₃H; SO₂R₆; SO₂NR₆R₇; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

Furthermore, the term "alkynyl" and "optionally substituted alkynyl" are used interchangeably herein and refer to any linear, branched, or cyclic alkyl or alkenyl with at least one carbon-carbon triple bond. Alkynyl chains may optionally be substituted by OH; NH₂; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR₆; NR⁶R⁷; CN; COR₆; CONR₆R₇; CO₂R⁶; C(S)OR₆; OCONR₆R₇;OCOR₆; OCO₂R₆; OC(S)OR₆; N₃; NO₂; NHCONR₆R₇; NHCOR₆; NR₆CO₂R₇; NHCO₂R₆; NHC(S)OR₆; NO₂; SR₆; SO₃H; SO₂R₆; SO₂NR₆R₇; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "aryl" as used herein is a group or part of a group being phenyl, naphthalenyl, anthracenyl, phenantrenyl, biphenyl, optionally fused to Het; each optionally substituted with one, two, three or four substituents selected from OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

The term "substituted" as used herein refers to a replacement of an atom or chemical group (e.g., H, NH₂, or OH) with a functional group, and particularly contemplated functional groups include nucleophilic groups (e.g., -NH₂, -OH, -SH, -NC, etc.), electrophilic groups (e.g., C(O)OR, C(X)OH, etc.), polar groups (e.g., -OH), non-polar groups (e.g., aryl, alkyl, alkenyl, alkynyl, etc.), ionic groups (e.g., -NH₃⁺), and halogens (e.g., -F, -CI), and all chemically reasonable combinations thereof. Thus, the term "functional group" and the term "substituent" are used interchangeably herein and refer to nucleophilic groups (e.g., -NH₂, -OH, -SH, -NC, - CN, etc.), electrophilic groups (e.g., C(O)OR, C(X)OH, C(Halogen)OR, etc.), polar groups (e.g., -OH), non-polar groups (e.g., aryl, alkyl, alkenyl, alkynyl, etc.), ionic groups (e.g., -NH₃⁺), and halogens.

Functional groups such as -OH, -NH₂, and the like, can incorporate protecting groups (abbreviated as PG) such as those known for those skilled in the art (GREENE'S PROTECTIVE. GROUPS IN ORGANIC. SYNTHESIS. Fourth Edition. PETER G. M. WUTS. and. THEODORA W. GREENE. 2007. Wiley-Interscience). By way of example, hydroxyl protection (Greene's vide supra, pages 16-366), including 1,2-diols could be in the form of ethers, esters, cyclic acetals, cyclic ketals, and silyl derivatives, such as, but not limited to, methyl ether, methoxymethyl ether, methylthiomethyl ether, *t*-butylthiomethyl ether, (phenyldimethylsislymethoxymethyl) ether, benzyloxymethyl ether, *p-*methoxybenzyloxymethyl ether, *p*-nitrobenzyloxymethyl ether, *o*-nitrobenzyzloxymethyl ether, (4-methoxyphenoxy)methyl ether, guaiacolmethyl ether, *t*-butoxymethyl ether, 4-pentenyloxymethyl ether, siloxymethyl ether, 2-methoxethoxymethyl ether, 2,2,2-trichloroethoxymethyl ether, bis(2-chloroethoxy)methyl ether, 2-(trimethylsilyl)ethoxymethyl ether, menthoxymethyl ether, tetrahydropyranyl ether, 3-bromotetrahydropyranyl ether, tetrahydrothiopyranyl ether, 1-methoxycyclohexyl ether, 4-methoxytetrahydropyranyl ether, 4-methoxytetrahydrothiopyranyl ether, 4-methoxytetrahydrothiopyranyl *S, S*-Dioxido ether, 1-[(2-chloro-4-methyl)phenyl]-4-methoxypiperidin-4-yl ether, 1,4-dioxan-2-yl ether, 1,4-dioxan-2-yl ether, tetrahydrothiofuranyl ether, 2,3,3a,4,5,6,7,7a-octahydro-7,8,8-trimethyl-4,7-methanobenzofuran-2-yl ether, 1-ethoxyethyl ether, 1-(2-chloroethoxy)ethyl ether, 1-hydroxyethyl ether, 2-bromoethyl ether, 1-[2-(trimethylsilyl)ethoxy]ethyl ether, 1-(2-cyanoethoxy)ethyl ether, prenyl ether, cynnamyl ether, propargyl ether, p-nitrophenyl ether, 1-methyl-1-methoxyethyl ether, 1-methyl-1-benzyloxyethyl ether, 1-methyl-1-1-benzyloxy-2-fluoroethyl ether, 2,2,2-trichloroethyl ether, 2-trimethylsilylethyl ether, 2-(phenylselenyl)ethyl ether, *t*-butyl ether, allyl ether, *p*-chlorophenyl ether, *p*-methoxyphenyl ether, 2,4-dinitrophenyl ether, benzyl ether, *p*-methoxylbenzyl ether, 3,4-dimethoxybenzyl ether, 2,6-dimethoxybenyzl, *o*-nitrobenzyl ether, *p*-nitrobenzyl ether, *p*-bromobenzyl ether, *p*-chlorobenzyl ether, 2,6-dichlorobenzyl ether, 2,4-dinitrobenzyl ether, fluorous benzyl ether, trimethylsilylxylyl ether, *p-*phenylbenzyl ether, cumyl ether, *p*-azidobenzyl ether, 2,6-difluorobenzyl ether, *p*-cyanobenzyl ether, *p*-phenylbenzyl ether, 2-picolyl ether, 4-picolyl ether, 3-methyl-2-picolyl *N*-oxido ether, diphenylmethyl ether, *p,p'*-dinitrobenzhydryl ether, 5-dibenzosuberyl ether, triphenylmethyl ether, α-naphtyldiphenylmethyl ether, *p*-methoxyphenyldiphenylmethyl ether, di(*p-*methoxyphenyl)phenylmethyl ether, tri(*p*-methoxyphenyl)phenylmethyl ether, 4-(4'-bromophenacyloxyphenyl)diphenylmethyl ether, 4,4',4"-tris(4,5-dichlorophthalimidophenyl)methyl ether, pentadienylnitrobenzyl, *p*-azidobenzyl ether, *p-*(methylsulfinyl)benzyl ether, 2-naphthylmethyl ether, 2-quinolinylmethyl ether, 1-pyrenylmethyl ether, 4-methoxydiphenylmethyl ether, 4-phenyldiphenylmethyl ether, α-naphthyldiphenylmethyl ether, *p*-methoxyphenyldiphenylmethyl ether, anthryl ether, 9-phenylthioxanthyl ether and the like; Silyl ethers such as trimethylsilyl ether, triethylsilyl ether, triisopropylsilyl ether, dimethylhexylsilyl ether, 2-norbornyldimethylsilyl ether, *t-*butyldimethylsilyl ether, *t*-butyldiphenylsilyl ether, tribenzylsilyl ether, tri-*p*-xylylsilyl ether, triphenylsilyl ether, diphenylmethylsilyl ether, di-*t*-butylmethylsilyl ether, bis(*t*-butyl)-1-pyrenylmethoxysilyl ether, tris(trimethylsilyl)silyl ether, (2-hidroxystyryl)dimethylsilyl ether, *t-*butoxydiphenylsilyl ether, 1,1,3,3-tetraisopropyl-3-[2-(tripheynlmethoxy)ethoxy]disiloxane-1-yl ether, fluorous silyl ether, and the like; Esters such as formate, benzoylformate, acetate, chloroacetate, dichloroacetate, trichloroacetate, trichloroacetimidate, trilfuoroacetate, methoxyacetate, triphenylmethoxyacetate, phenoxyacetate, *p*-chloropheynyacetate, phenylacetate, diphenylacetate, 3-phenylpropionate, bisfluorous chain type propanoyl ester, 4-pentenoate, levulinate, pivaloate, adamantoate, crotonate, 4-methoxylcrotonate, benzoate, *p*-phenylbenzoate, mesitoate, 4-bromobenzoate, 2,5-diflourobenzoate, p-nitrobenzoate, picolinate, nicotinate, 2-(azidomethyl)benzoate, 4-azidobutirate, (2-azidomethyl)phenylacetate, 2-{[(tritylthio)oxy]methyl}benzoate, 2-(allyloxy)phenylacetate, 2-(prenyloxymethyl)benzoate, 4-benzyloxybutyrate, 4-trialkylsiloxybutyrate, 4-acetoxy-2,2-dimethylbutyrate, 2,2-dimethyl-4-pentanoate, 2-iodobenzoate, 4-nitro-4-methylpentanoate, o-(dibromomethyl)benzoate, 2-formylbenzenesulfonate, 4-(methylthiomethoxy)butyrate, 2-(methylthiomethoxymethyl)benzoate, 2-(chloroacetoxymethyl)benzoate, 2-[(2-chloracetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, chlorodiphenylacetate, isobutyrate, monosuccionate, tigloate, *o*-(methoxycarbonyl)benzoate, *p*-benzoate, α-napthoate, nitrate, alkyl *N,N,N',N'*-tetramethylphosphorodiamidate, 2-chlorobenzoate, and the like; Sulfonates such as sulfate, allylsulfonate, methanesulfonate, benzylsulfonate, tosylate, 2-trifluoromethylsulfonate and the like; Carbonates such as alkyl methyl carbonate, methoxymethyl carbonate, 9-fluoromethyl carbonate, ethyl carbonate, bromoethyl carbonate, 2-(trimethylsilyl)ethyl carbonate, isobutyl carbonate, *t*-butyl carbonate, vinyl carbonate, allyl carbonate, propargyl carbonate, *p*-nitrophenyl carbonate, benzyl carbonate, 2-dansylethyl carbonate, phenacyl carbonate, methyl dithiocarbonate, S-benzyl thiocarbonate and the like; Carbamates such as dimethylthiocarbamate, *N*-phenylcarbamate, and the like; Cyclic acetals and ketals such as methylene acetal, ethylidene acetal, *t*-butylmethylidene acetal, 1-*t-*butylethylidine ketal, 1-phenyethylidene ketal, 2-(methoxycarbonyl)ethylidene acetal, 2-(*t-*butylcarbonyl)ethylidene acetal, phenylsulfonylethylidene acetal, 3-(benzyloxy)propylidene acetal, isopropylidene acetal or acetonide, cyclopentylidene acetal, benzylidene acetal, *p-*methoxybenzylidene acetal, mesitylene acetal, naphthaldehyde acetal, 9-anthracene acetal, benzophenone ketal and the like; Chiral ketones such as camphor ketal, menthone ketal and the like; Cyclic ortho esters such as methoxymethylene acetal, ethoxymethylene acetal, dimethoxymethylene orto ester, methylidene orto ester, phthalide orto ester, 1,2-dimethoxyethylidene orto ester, 2-oxacyclopentylidene orto ester, butane 2,3-bisacetal, cyclohexane-1,2-diacetal, dispiroketals and the like; Silyl derivatives such as di-*t*-butylsilylene group, diakkylsilylene group, 1,3-(1,1,3,3-tetraisopropyldisiloxanylidene) derivative, 1,1,3,3-tetra-*t*-butoxydisiloxanylidene derivative, methylene-bis-(diisopropylsilanoxanylidene, 1,1,4,4-tetrapheynyl-1,4-disilanylidene, *o*-xylyl ether, 3,3'-oxybis(dimethoxytrityl)ether, and the like; cyclic carbonates; cyclic borate such as methyl boronate, ethyl boronate, and the like.

The term "optionally substituted" when referring to alkyl, alkenyl, alkynyl, cycloalkyl, cycloalkenyl, aryl and heterocycle is intended to cover groups having oxo, ethylenedioxy, alkanoyloxy, alkoxy, alkylthio, carboxyl, halogen, thienyl, acetyl, 1-oxopropyl, 2-oxopropyl, 2-oxobutyl, 3-oxobutyl, 3-oxopentyl, 4-oxopentyl, 4-oxohexyl, 5-oxohexyl, ethylenedioxymethyl, 1,1-ethylenedioxyethyl, 2,2-ethylenedioxyethyl, 1,1-ethylenedioxypropyl, 2,2-ethylenedioxypropyl, 3,3-ethylenedioxypropyl, 1,1-ethylenedioxybutyl, 2,2-ethylenedioxybutyl, 3,3-ethylenedioxybutyl, 4,4-ethylenedioxybutyl, 3,3-ethylenedioxypentyl, 4,4-ethylenedioxyhexyl, 5,5-ethylenedioxyhexyl, acetyloxymethyl, 2-acetyloxyethyl, 3-acetyloxypropyl, 3-acetyloxybutyl, 4-acetyloxybutyl, 3-propionyloxybutyl, 3-butyryloxybutyl, 3-valeryloxypentyl, 3-hexanoyloxyhexyl, 4-acetyloxypentyl, 5-acetyloxypentyl, 4-acetyloxyhexyl, 5-acetyloxyhexyl, 6-acetyloxyhexyl, methoxymethyl, ethoxymethyl, propoxymethyl, butoxymethyl, pentyloxymethyl, hexyloxymethyl, 1-methoxyethyl, 2-methoxyethyl, 2-ethoxyethyl, 2-propoxyethyl, 2-butoxyethyl, 2-pentyloxyethyl, 2-hexyloxyethyl, 3-methoxypropyl, 3-ethoxypropyl, 2-methoxybutyl, 4-ethoxybutyl, 3-methoxypentyl, 5-ethoxypentyl, 4-methoxyhexyl, 6-ethoxyhexyl, methylthiomethyl, ethylthiomethyl, propylthiomethyl, butylthiomethyl, pentylthiomethyl, hexylthiomethyl, 1-methylthioethyl, 2-methylthioethyl, 2-ethylthioethyl, 2-methylthiopropyl, 3-methylthiopropyl, 3-ethylthiobutyl, 4-butylthiobutyl, 5-methylthiopentyl, 6-ethylthiohexyl, carboxymethyl, 1-carboxyethyl, 2-carboxyethyl, 2-carboxypropyl, 3-carboxypropyl, 4-carboxybutyl, 5-carboxypentyl, 6-carboxyhexyl, fluoromethyl, bromomethyl, chloromethyl, iodomethyl, 2-chloroethyl, 2-bromopropyl, 3-iodopropyl, 4-fluorobutyl, 5-chloropentyl, 6-bromohexyl, 2-thienylmethyl, 1-(2-thienyl)ethyl, 2-(2-thienyl)ethyl and the like.

The term "amino acid" refers to any of a class of organic compounds that contains at least one amino group, -NH-, and one carboxyl group, -COOH. These compounds can be the natural amino acids present in peptides or can contain any substitution in the amino group, in the carboxyl group or in the side chain. They can also present different chirality of the peptidic natural amino acids or can have different backbone, linear or cyclic, but must present, as said, at least one amino group and one carboxyl group. Amino acids can incorporate functional or protecting groups, such as those known for those skilled in the art (T.W.Greene, *vide supra*)*.* Preferred amino acids include, but are not limited to, alanine, valine, leucine and isoleucine.

Preferably, organic solvent is selected from methanol, ethanol, propanol, isopropanol, t-butanol, n-butanol, ethyl acetate, isopropyl acetate, butyl acetate, dichloromethane, toluene, tetrahydrofuran, 2-methyltetrahydrofuran, acetonitrile, acetone, cyclopentyl methyl ether, methyl ethyl ketone, methyl isobutyl ketone, dimethylamide, dimethylformamide and dimethylsulfoxide.

The present invention also relates to the use of the compound of formula (I), according to any of the above embodiments for said compound, as a calcineurin inhibitor.

As mentioned above, calcineurin (CN or PPP3, formerly PP2B) is a serine/threonine protein phosphatase regulated by Ca2+ and calmodulin. CN regulates development, memory, cardiac function, and immune response by dephosphorylating a variety of protein substrates, including the family of cytosolic Nuclear Factor of Activated T-cells (NFATc) transcription factors. NFATc proteins have been shown to be direct substrates of calcineurin. Dephosphorylated NFATc proteins translocate into the nucleus where, in cooperation with other transcription factors, induce the expression of many cytokine and chemokine genes, leading to T cell activation. It also plays a prominent role in many physiological processes, including homeostasis, angiogenesis, myogenesis, adipogenesis, osteogenesis, chondrocyte differentiation, cardiovascular system development, pancreatic β-cell proliferation, hair follicle cell differentiation and remodelling, and activities of cells of the immune and nervous systems, including schizophrenia, diabetes mellitus, Down synthdrome, cardiac hypertrophy, as well as in neurodegenerative diseases, such as Alzheimer's disease, psychotic disorders, epilepsy, among others.

The present invention further relates to the use of the compound of formula (I), according to any of the above embodiments for said compound, as an inhibitor of the calciurenin-NFAT signaling pathway.

NFAT proteins are expressed in immune cells and play a key role in eliciting immune responses. The NFAT proteins are activated by calcineurin, and the activated NFAT proteins, in turn, induce transcription of cytokine genes which are required for an immune response. By NFAT or NFATc protein (nuclear factor of activated T cells) is meant a member of a family of transcription factors comprising the members NFAT1, NFAT2, NFAT3 and NFAT4, with several isoforms. Any other NFATc protein whose activation is calcineurin dependent is also meant to be included herien. NFATc proteins can be, e.g., mammalian proteins, e.g., human or murine. NFAT1, NFAT2 and NFAT4 are expressed in immune cells, e.g., T lymphocytes, and play a role in eliciting immune responses. NFATc proteins are involved in the transcriptional regulation of cytokine genes, e.g., IL-2, IL-3, IL-4, TNF-α and IFN-γ, during the immune response.

In view of the need in the state of the art for drugs that inhibit or prevent activity of the immune system, the present invention further relates to the use of the compound of formula (I), according to any of the above embodiments for said compound, as an immunosuppresive drug.

Accordingly, the present invention relates to an immunosuppressive agent that inhibits interaction between calcineurin and NFAT with reduced toxic effects, by selectively inhibiting the interaction between calcineurin and NFAT, without inhibiting the enzymatic activity of calcineurin for its other substrates. Accordingly, these immunosuppressive agent exhibits reduced side effects in comparison to the gold standard treatments using cyclosporine A or tacrolimus.

Compounds of formula (I) do not disrupt CN-NFATc interaction by the PxIxIT motif, although compounds of formula (I) strongly inhibit NFTAc activation in human HEK293T cells at several concentrations, *i.e.* compounds of formula (I) behave as potent calcineurin inhibitors useful as immunosuppressive drugs.

Furthermore, compounds of formula (I) do not affect the phosphatase activity of CNA towards pNPP or RII substrates, *i.e.* compounds of formula (I) avoid the severe side effects associated to current immunosuppressive drugs such as cyclosporine or tacrolimus that bind to calcineurin through its catalytic site.

To sum up, compounds of formula (I) exhibit a more efficient and selective activity as calcineurin inhibitors than current cornerstone drugs such as cyclosporine and tacrolimus.

Consequently, the present invention also relates to the compound of formula (I), according to any of the above embodiments for said compound, for use in the prevention or treatment of an autoimmune disease or organ transplant rejection. Preferably, said autoimmune disease is selected from arthritis, anemia, psoriasis, dermatitis, urticaria, sclerosis, multiple sclerosis, inflammatory bowel disease, Crohn's disease, colitis, endocarditis, endometriosis, episcleritis, respiratory distress syndrome, meningitis, iritis, choroiditis, spondylitis, sudden hering loss, rhinitis, encephalitis, uveitis, autoimmune mocarditis, leukocyte adhesion deficiency, lupus, systemic lupus erythemotosus, diabetis mellitus, tuberculosis, sarcoidosis, granulomatosis, vasculitides, autoimmune aplastic anemia, autoimmune neutropenia, CNS inflammatory disorders, Bechet's or Behcet's disease, pemphigoid, pemphigus, Reiter's disease, nephritis, IgM polyneuropathies, thrombocytopenia, hypothyroidism, hypoparathyroidism, thyroiditis, Grave's disease, polyglandular syndromes, paraneoplastic syndromes, encephalomyelitis, myasthenia gravis, autoimmune hepatitis, Guillain-Barre syndrome, Berger's disease, dermatosis, Celiac disease, amylotrophic lateral sclerosis, coronary artery disease, autoimmune hearing loss, polychondritis, paraneoplasic syndrome, channelopathies, epilepsy, migraine, autism, fibromyalgia, multiple endocrine failure, presenile dementia, Chagas' disease, rheumatic fever, recurrent abortion, erythema multiforme, alveolitis, leprosy, malaria, leishmaniasis, dengue, endocarditis, fibrosis, cyclitis, human immunodeficiency virus (HIV) infection, Alzheimer's disease, Epstein-Barr virus infection, keratoconjuctivitis, bronchitis, chronic obstructive airway disease, idiophatic facial paralysis, chronic fatigue syndrome, febris rheumatica, hypogonadism, pancreatitis, vitiligo, acquire immune deficiency syndrome, multiple organ injury syndrome, autoimmune atrophic gastritis, rheumatic diseases, myocarditis, nephrotic syndrome, sinusitis, eosinophilia, hyperalgesia, meningitis, diabetic disorders, valvulitis. More preferably, said autoimmune disease is selected from arthritis, anemia, psoriasis, dermatitis, sclerosis, multiple sclerosis, meningitis, rhinitis, lupus, diabetis mellitus, CNS inflammatory disorders, pemphigus, nephritis, autoimmune hepatitis, Celiac disease, migraine, fibromyalgia, endocarditis, coronary artery disease, epilepsy, Chagas' disease, rheumatic fever, malaria, Alzheimer's disease, Epstein-Barr virus infection, bronchitis, chronic obstructive airway disease, chronic fatigue syndrome, pancreatitis, acquire immune deficiency syndrome, multiple organ injury syndrome, myocarditis, meningitis, diabetic disorders.

The compounds of the present invention may be prepared according to the procedures described hereinafter, which are meant to be applicable for as well the racemates, stereochemically pure intermediates or end products, or any stereoisomeric mixtures. The racemates or stereochemical mixtures may be separated into stereoisomeric forms at any stage of the synthesis procedures.

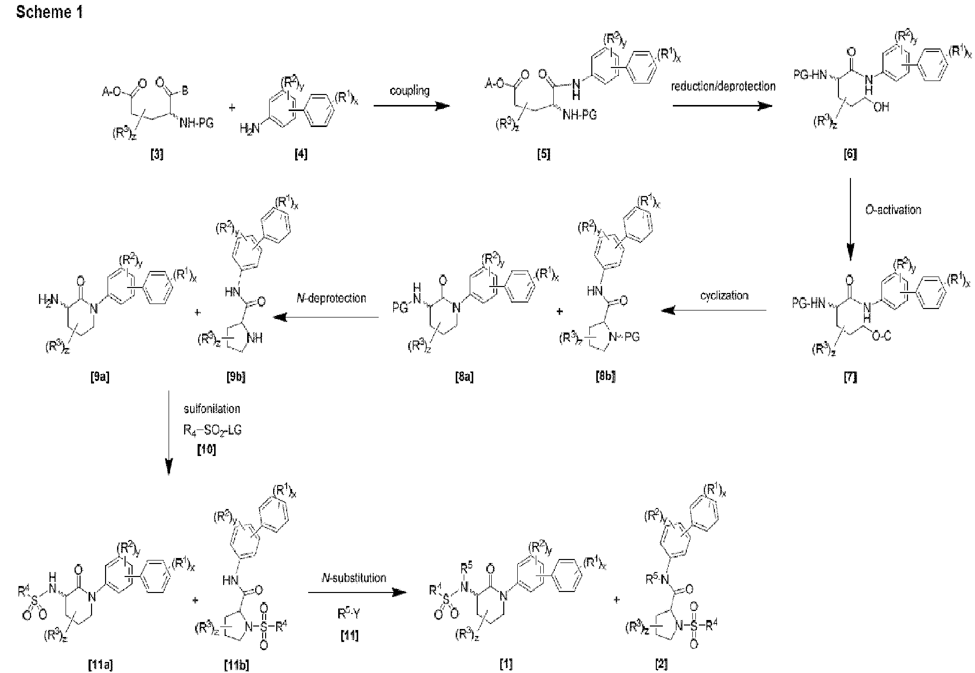

As shown in the above scheme 1, coupling of a compound of formula [3] with the primary amine compound of formula [4] gives the amide derivative compound of formula [5]. The coupling reaction occurs in an organic solvent, such as a chlorinated solvent, preferably dichloromethane, 1,2-dichloroethane or chloroform, at a temperature preferably between -70° C and 40°C, more preferably between -10° C and 25° C. Compound of formula [5] comprises a group -CO₂-A in the form of an activated carboxyl derivative, such as acid chlorides, anhydrides, or active esters such as O-acylisoureas or acyloxyphosphonium derivatives. In a particular embodiment the carbonyl compound is carboxylic acid, the carboxyl activate derivative part of form A is O-acylisourea and the activating group is a carbodiimide coupling reagent such as dicyclohexylcarbodiimide (DCC), while in another the coupling group is diisopropylcarbodiimide (DIPC).

The corresponding reduction or deprotection reaction of compound [5] yields the alcohol of formula [6]. In a particular embodiment, PG (protecting group) is a benzyl protecting group, and the deprotection reaction comprises the chemoselective reduction of the metal hydride with a reductive agent such as NaBH₄ or Ca(BH₄)₂ in a polar protic solvent, such as ethanol or 2-propanol at a temperature preferably between -10° C and 25°C, more preferably between 0° C and 10° C.

The activation of compound [6] to furnish compound of formula [7] (C meaning activating group) occurs by means of sulfonyl halides, preferably para-toluenesulfonyl halides, methanesulfonyl halides or trifluoromethanesulfonyl halides, in the presence of an organic aliphatic or aromatic base, such as pyridine, imidazole, or triethylamine. In a particular embodiment, C group is a methanesulfonyl activating group, and the reaction occurs in a chlorinated solvent, preferably dichloromethane, 1,2-dichloroethane or chloroform, in anhydrous or non anhydrous conditions, at a temperature preferably between -10° C and 40°C, more preferably between 0° C and 25° C.

Treatment of compound [7] under cyclisation conditions yields the lactam compound of formula [8a] and the pyrrolidine compound of formula [8b]. The reaction occurs in the presence of an inorganic or organic base, such as sodium hydride, potassium *tert*-butoxide or lithium diisopropylamide, at a temperature preferably between -78°C and 60°C, more preferably between -40°C and 0°C. The reaction solvent is a polar aprotic solvent, preferably acetonitrile, tetrahydrofuran, dimethylformamide, or dimethylsulfoxide.

The *N*-deprotection of compounds [8a] and [8b] yields compounds of formula [9a] and [9b], respectively, where PG is an amino protecting group, carbamate, urea-type derivative, amide, cyclic imide, alkyl, aryl, imine, enamine or heteroatom. In a particular embodiment, the protecting group is *tert*-butoxycarbonyl group and the deprotecting agent is trifluoroacetic acid in a chlorinated solvent, preferably dichloromethane, 1,2-dichloroethane or chloroform, at a trifluoroacetic acid composition preferably between 5% and 90%, more preferably between 15% and 70%, at a temperature preferably between 0°C and 45°C, more preferably between 10°C and 30°C.

The substitution reaction of [9a] or [9b] with compounds of formula R⁴-SO₂-LG, where LG means "leaving group", being said LG group preferably an halogen atom, more preferably bromine or chlorine, yields the corresponding substituted sulfonamides of formula [11a] and [11b], respectively. The reaction solvent is a chlorinated solvent, preferably dichloromethane, 1,2-dichloroethane or chloroform, or a polar aprotic solvent, preferably acetonitrile, tetrahydrofuran, or dimethylformamide, at a temperature preferably between 0° C and 40°C, more preferably between 10° C and 25° C.

Under substitution or coupling conditions with compounds of formula R⁵-Y [11], wherein Y means "leaving group" in substitution reaction and "activating group" in coupling reactions, being said Y preferably is a halogen atom, more preferably bromine or chlorine in substitution reaction, or an activated carboxyl derivative in coupling reactions, compounds [11a] and [11b] are converted to the final compounds of formula [1] and [2], respectively. The reaction solvent is a hydrous or anhydrous polar aprotic solvent, preferably acetonitrile, tetrahydrofuran, or dimethylformamide, at a temperature preferably between -78°C and 60°C, more preferably between -78°C and 25°C.

Both racemic as well as pure enantiomers of [1] and [2] can be accessed by this approach depending on the stereochemical integrity of the starting material.

Scheme 1 does not limit the methods of obtantion of compounds of formula (I), which could alternatively be prepared by a different synthetic approach in which after coupling of compounds [3] and [4], *N*-deprotection and subsequent sulfonylation render the corresponding intermediate that can be further deprotected and activated before cyclation.

The process according to present invention may also include isolation and/or purification steps of each intermediate or final product produced by standard operation means selected from chromatography, precipitation, filtration, concentration and crystallization.

The present invention also relates to novel compounds represented by formula (I) (see Tables 1 and 2).

**Table 1**

| **CODE** | **R1** | **R2** | **R3** | **R4** | **R5** | **n** | **p** | **q** |
|---|---|---|---|---|---|---|---|---|
| IUCT-0460 | F | 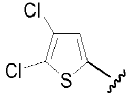 | H | H | OMe | 1 | 1 | 1 |
| IUCT-0461 | F | 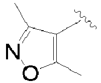 | H | H | OMe | 1 | 1 | 1 |
| IUCT-0462 | F | 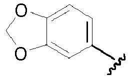 | H | H | OMe | 1 | 1 | 1 |
| IUCT-0463 | F | 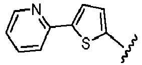 | H | H | OMe | 1 | 1 | 1 |
| IUCT-0464 | F | 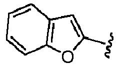 | H | H | OMe | 1 | 1 | 1 |
| IUCT-0465 | F | 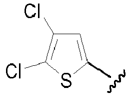 | CH₂CN | H | OMe | 1 | 1 | 1 |
| IUCT-0466 | F | 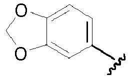 | CH₂CO₂Et | H | OMe | 1 | 1 | 1 |
| IUCT-0467 | F | 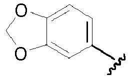 | 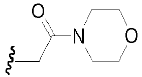 | H | OMe | 1 | 1 | 1 |
| IUCT-0468 | F | 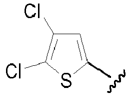 | CH₂CONH₂ | H | OMe | 1 | 1 | 1 |
| IUCT-0469 | F | 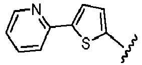 | CH₂CO₂Et | H | OMe | 1 | 1 | 1 |
| IUCT-0463.01 | F | 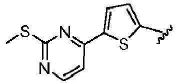 | H | H | OMe | 1 | 1 | 1 |
| IUCT-0463.02 | F | 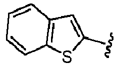 | H | H | OMe | 1 | 1 | 1 |
| IUCT-0463.03 | F | 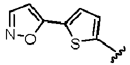 | H | H | OMe | 1 | 1 | 1 |
| IUCT-0463.04 | F | 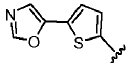 | H | H | OMe | 1 | 1 | 1 |
| IUCT-0463.05 | F | 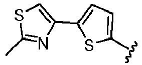 | H | H | OMe | 1 | 1 | 1 |
| IUCT-0463.06 | F | 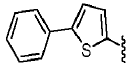 | H | H | OMe | 1 | 1 | 1 |

**Table 2**

| CODE | IUPAC NAME |
|---|---|
| IUCT-0460 | 4,5-Dichloro-N-(1-(3-fluoro-2'-methoxy-[1,1'-biphenyl]-4-yl)-2-oxopiperidin-3-yl)thiophene-2-sulfonamide |
| IUCT-0461 | N-(1-(3-fluoro-2'-methoxy-[1,1'-biphenyl]-4-yl)-2-oxopiperidin-3-yl)-3,5-dimethylisoxazole-4-sulfonamide |
| IUCT-0462 | N-(1-(3-fluoro-2'-methoxy-[1,1'-biphenyl]-4-yl)-2-oxopiperidin-3-yl)benzo[d][1,3]dioxole-5-sulfonamide |
| IUCT-0463 | N-(1-(3-fluoro-2'-methoxy-[1,1'-biphenyl]-4-yl)-2-oxopiperidin-3-yl)-5-(pyridin-2-yl)thiophene-2-sulfonamide |
| IUCT-0464 | N-(1-(3-fluoro-2'-methoxy-[1,1'-biphenyl]-4-yl)-2-oxopiperidin-3-yl)benzofuran-2-sulfonamide |
| IUCT-0465 | 4,5-Dichloro-N-(cyanomethyl)-N-(1-(3-fluoro-2'-methoxy-[1,1'-biphenyl]-4-yl)-2-oxopiperidin-3-yl)thiophene-2-sulfonamide |
| IUCT-0466 | Ethyl N-(benzo[d][1,3]dioxol-5-ylsulfonyl)-N-(1-(3-fluoro-2'-methoxy-[1,1'-biphenyl]-4-yl)-2-oxopiperidin-3-yl)glycinate |
| IUCT-0467 | N-(1-(3-fluoro-2'-methoxy-[1,1'-biphenyl]-4-yl)-2-oxopiperidin-3-yl)-N-(2-morpholino-2-oxoethyl)benzo[d][1,3]dioxole-5-sulfonamide |
| IUCT-0468 | 2-((4,5-dichloro-N-(1-(3-fluoro-2'-methoxy-[1,1'-biphenyl]-4-yl)-2-oxopiperidin-3-yl)thiophene)-2-sulfonamido)acetamide |
| IUCT-0469 | Ethyl N-(1-(3-fluoro-2'-methoxy-[1,1'-biphenyl]-4-yl)-2-oxopiperidin-3-yl)-N-((5-(pyridin-2-yl)thiophen-2-yl)sulfonyl)glycinate |
| IUCT-0463.01 | N-(1-(3-fluoro-2'-methoxy-[1,1'-biphenyl]-4-yl)-2-oxopiperidin-3-yl)-5-(2-(methylthio)pyrimidin-4-yl)thiophene-2-sulfonamide |
| IUCT-0463.02 | N-(1-(3-fluoro-2'-methoxy-[1,1'-biphenyl]-4-yl)-2-oxopiperidin-3-yl)benzo[b]thiophene-2-sulfonamide |
| IUCT-0463.03 | N-(1-(3-fluoro-2'-methoxy-[1,1'-biphenyl]-4-yl)-2-oxopiperidin-3-yl)-5-(isoxazol-5-yl)thiophene-2-sulfonamide |
| IUCT-0463.04 | N-(1-(3-fluoro-2'-methoxy-[1,1'-biphenyl]-4-yl)-2-oxopiperidin-3-yl)-5-(oxazol-5-yl)thiophene-2-sulfonamide |
| IUCT-0463.05 | N-(1-(3-fluoro-2'-methoxy-[1,1'-biphenyl]-4-yl)-2-oxopiperidin-3-yl)-5-(2-methylthiazol-4-yl)thiophene-2-sulfonamide |
| IUCT-0463.06 | N-(1-(3-fluoro-2'-methoxy-[1,1'-biphenyl]-4-yl)-2-oxopiperidin-3-yl)-5-phenylthiophene-2-sulfonamide |

The present invention will be further described by means of examples which do not intend to limit the scope of the instant invention. Comparative examples are also provided. The reaction schemes provided below only intend to illustrate how to obtain various compounds as disclosed herein. As it is well known by a person skilled in the art, different conditions can be applied, when necessary, providing that the the general process as disclosed herein is performed.

The following Examples are offered for illustrative purposes only and are not intended to limit the scope of the present invention in any way.

### EXAMPLES

### Comparative example 1: Preparation of intermediate [5]: Benzyl 4-((tert-butoxycarbonyl)amino)-5-((3-fluoro-2'-methoxy-[1,1'-bipheny]1-4-yl)amino)-5-oxopentanoate (IUCT-0451)

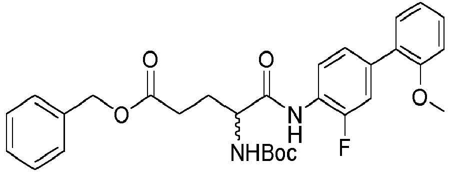

To a stirred solution of Boc-L-glutamic acid 5-benzyl ester **[3]** (0.768 g, 2.28 mmol) in anhydrous CH₂Cl₂ (3.5 mL) at 0°C, a solution of DCC (0.566 g, 2.74 mmol) in anhydrous CH₂Cl₂ (2.5 mL) was added dropwise. The resulting white slurry was sonicated and extra anhydrous CH₂Cl₂ (1 mL) was added. After 15 minutes, anhydrous 3-fluoro-2'-methoxy-[1,1'-biphenyl]-4-amine (0.5 g, 2.3 mmol) dissolved in anhydrous CH₂Cl₂ (1.5 mL) was added dropwise to the reaction mixture over 10 minutes at 0°C. The suspension turns orange. The mixture was stirred 15 minutes at 0°C and at room temperature until total consumption of starting materials (from 4 to 24 hours) and filtered through Celite® to remove insoluble material. The resulting liquid was evaporated to dryness and chromatographically purified on alumina using Hexane/EtOAc 80/20, yielding the desired product (1.044 g, 83%).
¹H-NMR (400 MHz, CDCl₃), δ: 8.44 (br, 1H, CONHPh), 8.21 (t, 1H, *J*=8.3 Hz, H_{Ar}), 7.45 (m, 1H, H_{Ar}), 7.35-7.20 (m, 8H, H_{Ar}), 6.94 (m, 2H, H_{Ar}), 5.27 (br, 1H, CHNHBoc), 5.09 (s, 2H, BnOCH₂), 4.28 (br, 1H, CH₂CHNHBoc), 3.75 (s, 3H, OCH₃), 2.65-2.40 (mc, 1H, 1xOCOCH₂CH₂), 2.25 (mc, 1H, 1xOCOCH₂CH₂), 1.99-1.90 (mc, 1H, 1x OCOCH₂CH₂), 1.40 (s, 9H, NHCO₂C(CH₃)₃) ppm.
MS-ESI (+): [M + Na]⁺ = 559
MS-ESI (-): [M - H]⁻ = 535

### Comparative example 2: Tert-butyl (1-((3-fluoro-2'-methoxy-[1,1'-bipheny]1-4-yl))amino)-5-hydroxy-1-oxopentan-2-yl)carbamate (ICUT-0456) Preparation of intermediate [6]:

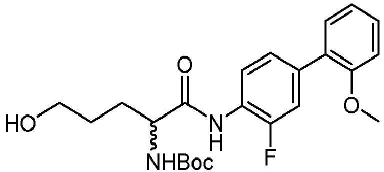

To a stirred suspension of NaBH₄ (0.26 g, 6.9 mmol) in 200 ml EtOH at 0°C was added crushed CaCl₂ (0.40 g, 3.45 mmol) in portions during 15 min. After that, compound **[5]** (0.93 g, 1.7 mmol) was added in portions during 10 minutes. The solution was stirred for 3.5 h, warming to room temperature. The crude was neutralized at 0°C using HCI 0.1 M, and the aqueous phase was extracted in AcOEt. The organic phase was washed using saturated NaCl, dried over anhydrous Na₂SO₄ and evaporated to dryness. The resulting oil residue was chromatographically purified over SiO₂ in Hexane/AcOEt (40:60), furnishing the desired product (0.48 g, 65%).
¹H-NMR (400 MHz, CDCl₃), δ: 8,60 (br, 1H, CONHPh), 8.27 (t, 1H, *J*₁=8.4 Hz, H_{Ar}), 7.30-7.20 (m, 4H, 4xH_{Ar}), 6.9 (m, 2H, 2xH_{Ar}), 5.2 (br, 1H, CHNHBoc), 4.45 (br, 1H, CHNHBoc), 3.75-3.70 (m, 5H, CH₂OH +OCH₃), 2.0-1.65 (m, 4H, CH₂CH₂), 1.40 (s, 9H, NHCO₂C(CH₃)₃) ppm.
MS-ESI (+): [M + Na]⁺ = 455
MS-ESI (-): [M - H]⁻ = 431

### Comparative example 3: Preparation of intermediate [7]: 4-((Tert-butoxycarbonyl)amino)-5-((3-fluoro-2'-methoxy-[1,1'-biphenyl]-4-yl)amino)-5-oxopentyl methanesulfonate (IUCT-0457)

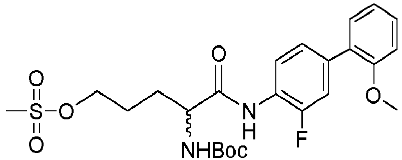

To a stirred solution of compound **[6]** (0.39 g, 0.90 mmol) in 6 mL anhydrous CH₂Cl₂ at 0°C, anhydrous Et₃N (0.19 mL, 1.35 mmol, 1.5 eq) was added. After 10 minutes, MsCI was added dropwise (0.08 mL, 1.08 mmol, 1.2 eq) at 0°C. The mixture was stirred for 10 minutes at 0°C and up to room temperature until total consumption of starting materials. After then, the crude was evaporated to dryness, and filtered over SiO₂ using AcOEt as the eluant, without further purification. The desired product was obtained accordingly (0.045 g, 99%).
¹H-NMR (400MHz, CDCl₃), δ: 8.33 (br, 1H, CONHPh), 8.20 (t, 1H, *J*₁=8.4 Hz, H_{Ar}), 7.30-7.15 (m, 4H, 4xH_{Ar}), 6.94 (m, 2H, 2xH_{Ar}), 5.07 (br, 1H, CHNHBoc), 4.45 (br, 1H, CHNHBoc), 3.70 (s, 5H, CH₂OMs +OCH₃), 2.99 (s, 3H, CH₃SO₂-), 2.0-1.65 (m, 4H, CH₂CH₂), 1.40 (s, 9H, NHCO₂C(CH₃)₃) ppm.
MS-ESI (+): [M + Na]⁺ = 533.1

### Comparative example 4: Preparation of Intermediate [8a]: Tert-butyl (1-(3-fluoro-2'-methoxy-[1,1'-biphenyl]-4-yl)-2-oxopiperidin-3-yl)carbamate (IUCT-0459)

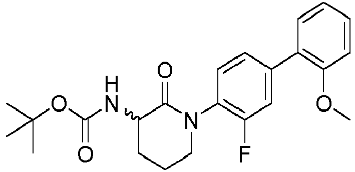

Under inert atmosphere, LDA 2M (1.25 mmol, 1.5 eq) was added dropwise to a solution of intermediate **[7]** (0.424 g, 0.83 mmol) in anhydrous DMF (9 mL) at -10°C. The solution was stirred at this temperature for 1 h, until total consumption of starting material. After then, the crude was evaporated to dryness, and purified over silica gel using Hexane/AcOEt from 70/30 to 50/50 as the eluant, yielding 0.29 g (84 %) of the desired product **[8a]** and 0.02 g (5 %) of the co-product **[8b].**

### Compound [8a]:

¹H-NMR (400MHz, CDCl₃): δ 7.32-7.2 (m, 4H_{Ar}), 6.95 (m, 3H_{Ar}), 5.5 (br, 1H, NHBoc), 4.24 (m, 1H, CHNHBoc), 3.76 (s, 3H, -OCH₃), 3.61 (m, 2H, -CHCH₂CH₂CH₂N-), 2.61 (m, 1H, -CH₂-), 2.04 (m, 2H, -CHCH₂CH₂CH₂N-), 1.68 (m, 1H, CHCH₂CH₂CH₂-), 1.40 (s, 9H, *^{t}*Bu) ppm.
MS-ESI (+): [M + Na]⁺ = 437.1

### Compound [8b]:

¹H-NMR (400MHz, CDCl₃): δ 9.4 (br, 1H, NHPh), 8.3 (t, 1H_{Ar}), 7.3 (m, 4H_{Ar}), 6.9 (m, 2H_{Ar}), 4.4 (br, 1H, CH), 3.76 (s, 3H, -OCH₃), 3.4 (br, 2H, CH₂), 1.93 (m, 2H, CH₂), 1.49 (s, 9H, *^{t}*Bu), 1.49 (s, 2H, CH₂) ppm.
MS-ESI (+): [M + Na]⁺ = 437.1

### Comparative example 5: Preparation of Intermediate [9a]: 3-amino-1-(3-fluoro-2'-methoxy-[1,1'-biphenyl]-4-yl)piperidin-2-one (trifluoroacetate salt)

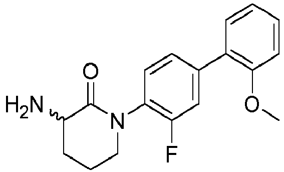

To a stirred solution of **[8a]** (0.057 g, 0.14 mmol) in 1.5 mL of CH₂Cl₂, trifluoroacetic acid (0.32 mL, 30 eq) was added at room temperature, and the mixture was sealed and stirred for 0.5 h. After then, the crude was evaporated to dryness, yielding an orange oily residue of the organic salt **[9a],** which was precipitated using *ⁱ*Pr₂O. The remaining solid was used without further purification.
MS-ESI (+): [M + Na]⁺ = 315

### Example 6: Synthesis of 4,5-dichloro-N-(1-(3-fluoro-2'-methoxy-[1,1'-biphenyl]-4-yl)-2-oxopiperidin-3-vl)thiophene-2-sulfonamide (IUCT-460):

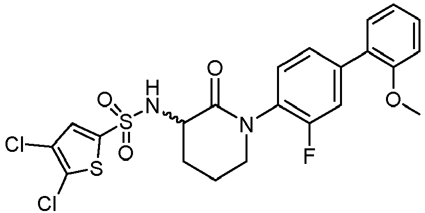

Under inert atmosphere, to a stirred solution of compound **[9a]** (0.085 g, 0.20 mmol) in 2.5 mL anhydrous CH₂Cl₂, anhydrous Et₃N (3.5 eq) was added at room temperature. This mixture was stirred for 15 min, and then sulfonyl halide **[10]** (1.05 eq) was slowly added in portions or dropwise. The reaction was stirred until total consumption of starting materials. Then, the solvent was removed and the crude was chromatographically purified over SiO₂ using Hexane/AcOEt 50/50 as the eluant, yielding 0.073 g (70%) of the desired product.
¹H-NMR (400MHz, CDCl₃): δ: 7.39 (s, 1H, H_{Het}), 7.35-7.1 (m, 5H_{Ar}), 6.94 (m, 2H, 2xH_{Ar}), 5.99 (br, 1H, CHNHSO₂), 3.8 (br, 1H, CHNHSO₂), 3.75 (s, 3H, OCH₃), 3.61 (m, 2H, - CHCH₂CH₂CH₂N-), 2.58 (m, 1H, -CHCH₂CH₂CH₂N-), 2.05 (m, 2H, -CHCH₂CH₂CH₂N-), 1.95 (m, 1H, -CHCH₂CH₂CH₂N-) ppm.
MS-ESI (+): [M + Na]⁺ = 552.2
MS-ESI (-): [M - H]⁻ = 527.0

### Example 7: Synthesis of N-(1-(3-fluoro-2'-methoxy-[1,1'-biphenyl]-4-yl)-2-oxopiperidin-3-yl)-3,5-dimethylisoxazole-4-sulfonamide (IUCT-0461)

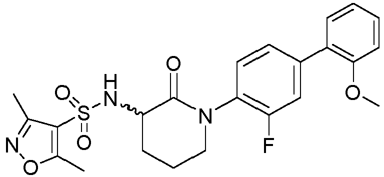

Following a procedure analogous to that described in Example 6, the title compound was obtained as a white solid in 45 % yield.
¹H-NMR (400MHz, CDCl₃), δ: 7.35-7.1 (m, 5H_{Ar}), 6.96 (m, 2H, 2xH_{Ar}), 5.94 (br, 1H, CHNHSO₂), 3.76 (s, 3H, OCH₃), 3.59 (br, 3H, CHNHSO₂ + -CHCH₂CH₂CH₂N-), 2.62 (s, 3H, CH_{3Het}), 2.53 (m, 1H, -CHCH₂CH₂CH₂N-), 2.41 (s, 3H, CH_{3Het}), 2.1-1.8 (m, 3H, 2H from -CHCH₂CH₂CH₂N-+ 1H from -CHCH₂CH₂CH₂N-) ppm.
MS-ESI (+): [M + Na]⁺ = 497.2

### Example 8: Synthesis of N-(1-(3-fluoro-2'-methoxy-[1,1'-biphenyl]-4-yl)-2-oxopiperidin-3-yl)benzo[d][1,3]dioxole-5-sulfonamide (IUCT-0462)

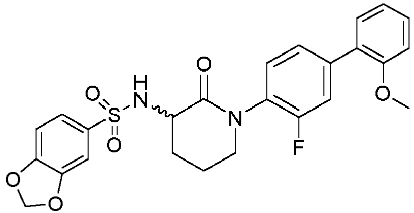

Following a procedure analogous to that described in Example 6, the title compound was obtained as a white solid in 80 % yield.
¹H-NMR (400MHz, CDCl₃), δ: 7.47 (m, 1H_{Ar}), 7.40-7.15 (m, 6H_{Ar}), 6.96 (m, 2H, 2xH_{Ar}), 6.75 (d, ³*J*=8 Hz, 2H_{Ar}), 5.96 (s, 2H, OCH₂O), 3.74 (s, 3H, OCH₃), 3.59 (br, 3H, CHNHSO₂ + - CHCH₂CH₂CH₂N-), 2.53 (m, 1H, -CHCH₂CH₂CH₂N-), 2.1-1.8 (m, 3H, 2H from - CHCH₂CH₂CH₂N-+ 1H from -CHCH₂CH₂CH₂N-) ppm.
MS-ESI (+): [M + Na]⁺ = 521.2
MS-ESI (-): [M - H]⁻ = 496.9

### Example 9: Synthesis of N-(1-(3-fluoro-2'-methoxy-[1,1'-biphenyl]-4-yl)-2-oxopiperidin-3-yl)-5-(pyridin-2-yl)thiophene-2-sulfonamide (IUCT-0463)

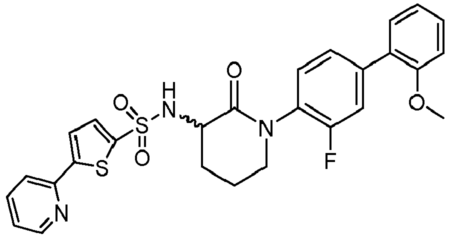

Following a procedure analogous to that described in Example 6, the title compound was obtained as a white solid in 57 % yield.
¹H-NMR (400MHz, CDCl₃), δ: 1H NMR (400 MHz, CDCl3) δ 8.66 (d, *J* = 4.0 Hz, 1H_{Het}), 7.77 (ddd, *J* = 15.9, 10.8, 5.9 Hz, 3H_{Ar}), 7.59 (d, *J* = 3.9 Hz, 1H_{Ar}), 7.51 - 7.15 (m, 6H_{Ar}), 7.15-6.96 (m, 2H_{Ar}), 6.14 (s, 1H, CHNHSO₂), 4.02 (d, *J* = 11.3 Hz, 1H), 3.87 (s, 3H, OCH₃), 3.71 (d, *J* = 5.4 Hz, 2H), 2.77 (d, *J* = 7.3 Hz, 1H), 2.13 (d, *J* = 18.2 Hz, 4H) ppm.
¹³C-NMR (100 MHz, CDCl₃), δ: 149.52, 136.68, 132.80, 130.50, 129.21, 127.91, 125.77, 123.73, 123.04, 120.81, 118.93, 117.55, 116.06, 61.48, 60.05, 55.39, 51.08, 48.15, 48.08, 47.99, 47.95, 28.78, 22.24, 14.22 ppm.
MS-ESI (+): [M + H]⁺ = 538.0; [M + Na]⁺ = 560.0
MS-ESI (-): [M - H]⁻ = 535.9

### Example 10: Synthesis of N-(1-(3-fluoro-2'-methoxy-[1,1'-biphenyl]-4-yl)-2-oxopiperidin-3-vl)benzofuran-2-sulfonamide (IUCT-0464)

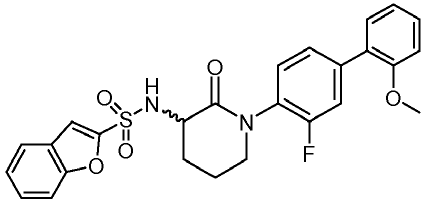

Following a procedure analogous to that described in Example 6, the title compound was obtained as a white solid in 70 % yield.
¹H-NMR (400MHz, CDCl₃), δ: 7.70-7.02 (m, 12H_{Ar}), 6.06 (s, 1H, CHNHSO₂), 3.76 (s, 3H, OCH₃), 3.59 (br, 3H, CHNHSO₂ + -CHCH₂CH₂CH₂N-), 2.53 (m, 1H, -CHCH₂CH₂CH₂N-), 2.41 (s, 3H, CH_{3Het}), 2.1-1.8 (m, 3H, 2H from -CHCH₂CH₂CH₂N-+ 1H from -CHCH₂CH₂CH₂N-) ppm.
MS-ESI (+): [M + Na]⁺ = 517.2

### Example 11: Synthesis of N-(1-(3-fluoro-2'-methoxy-[1,1'-biphenyl]-4-yl)-2-oxopiperidin-3-yl)-5-(2-(methylthio)pyrimidin-4-yl)thiophene-2-sulfonamide (IUCT-0463.01)

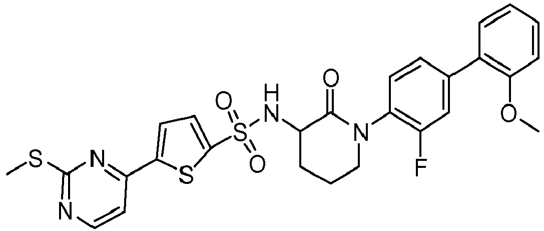

Following a procedure analogous to that described in Example 6, the title compound was obtained as a white solid in 43% yield.
¹H-NMR (400MHz, CDCl₃), δ: 9.14 (d, ³*J* = 7.5 Hz, 1H_{Het}), 8.5 (br, 1H, CHNHSO₂), 7.90-7.40 (m, 7H_{Ar} + 1H_{Het}), 6.96 (m, 2H, 2xH_{Ar}), 3.76 (s, 3H, OCH₃), 3.59 (br, 3H, CHNHSO₂ + - CHCH₂CH₂CH₂N-), 2.60 (s, 3H, SCH₃), 2.53 (m, 1H, -CHCH₂CH₂CH₂N-), 2.1-1.8 (m, 3H, 2H from -CHCH₂CH₂CH₂N-+ 1H from -CHCH₂CH₂CH₂N-) ppm.
MS-ESI (+): [M + Na]⁺ = 611.1

### Example 12: Synthesis of N-(1-(3-fluoro-2'-methoxy-[1,1'-biphenyl]-4-yl)-2-oxopiperidin-3-yl)benzo[b]thiophene-2-sulfonamide (IUCT-0463.02)

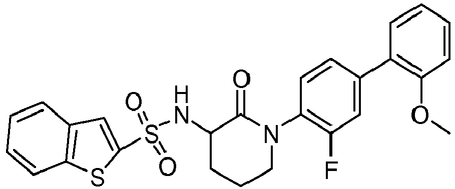

Following a procedure analogous to that described in Example 6, the title compound was obtained as a white solid in 64% yield.
¹H-NMR (400MHz, CDCl₃), δ: 8.5 (br, 1H, CHNHSO₂), 7.90-7.50 (m, 10H_{Ar}), 7.02 (m, 2H, 2xH_{Ar}), 3.76 (s, 3H, OCH₃), 3.59 (br, 3H, CHNHSO₂ + -CHCH₂CH₂CH₂N-), 2.60 (s, 3H, SCH₃), 2.53 (m, 1H, -CHCH₂CH₂CH₂N-), 2.1-1.8 (m, 3H, 2H from -CHCH₂CH₂CH₂N-+ 1H from - CHCH₂CH₂CH₂N-) ppm.
MS-ESI (+): [M + Na]⁺ = 533.2

### Example 13: Synthesis of N-(1-(3-fluoro-2'-methoxy-[1,1'-biphenyl]-4-yl)-2-oxopiperidin-3-yl)-5-(isoxazol-5-yl)thiophene-2-sulfonamide (IUCT-0463.03)

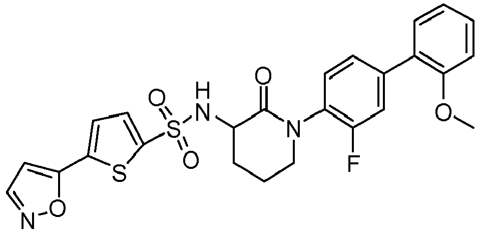

Following a procedure analogous to that described in Example 6, the title compound was obtained as a white solid in 68% yield.
¹H-NMR (400MHz, CDCl₃), δ: 8.5 (br, 1H, CHNHSO₂), 8.11 (d, ³*J* = 7.5 Hz, 1H_{Het}), 7.80-7.00 (m, 7H_{Ar}), 7.02 (m, 2H, 2xH_{Ar}), 6.65 (d, ³*J* = 7.5 Hz, 1H_{Het}), 3.76 (s, 3H, OCH₃), 3.59 (br, 3H, CHNHSO₂ + -CHCH₂CH₂CH₂N-), 2.53 (m, 1H, -CHCH₂CH₂CH₂N-), 2.1-1.8 (m, 3H, 2H from - CHCH₂CH₂CH₂N-+ 1H from -CHCH₂CH₂CH₂N-) ppm.
MS-ESI (+): [M + Na]⁺ = 550.4

### Example 14: Synthesis of N-(1-(3-fluoro-2'-methoxy-[1,1'-biphenyl]-4-yl)-2-oxopiperidin-3-yl)-5-(oxazol-5-yl)thiophene-2-sulfonamide (IUCT-0463.04)

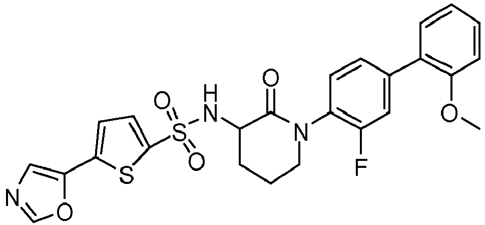

Following a procedure analogous to that described in Example 6, the title compound was obtained as a white solid in 61% yield.
¹H-NMR (400MHz, CDCl₃), δ: 8.5 (br, 1H, CHNHSO₂), 8.11 (m, 2H, 1H_{Het}+1H_{Ar}), 7.80-7.00 (m, 6H_{Ar}), 7.02 (m, 3H, 1H_{Het}+2H_{Ar}), 3.76 (s, 3H, OCH₃), 3.59 (br, 3H, CHNHSO₂ + - CHCH₂CH₂CH₂N-), 2.53 (m, 1H, -CHCH₂CH₂CH₂N-), 2.1-1.8 (m, 3H, 2H from - CHCH₂CH₂CH₂N-+ 1H from -CHCH₂CH₂CH₂N-) ppm.
MS-ESI (+): [M + Na]⁺ = 550.4

### Example 14: Synthesis of N-(1-(3-fluoro-2'-methoxy-[1,1'-biphenyl]-4-yl)-2-oxopiperidin-3-yl)-5-(2-methylthiazol-4-yl)thiophene-2-sulfonamide (IUCT-0463.05)

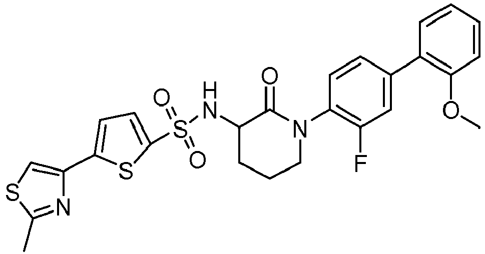

Following a procedure analogous to that described in Example 6, the title compound was obtained as a white solid in 55% yield.
¹H-NMR (400MHz, CDCl₃), δ: 8.5 (br, 1H, CHNHSO₂), 8.0-7.8 (m, 3H, 2H_{Het}+1H_{Ar}), 7.5-7.3 (m, 7H_{Ar}), 3.76 (s, 3H, OCH₃), 3.59 (br, 3H, CHNHSO₂ + -CHCH₂CH₂CH₂N-), 2.76 (s, 3H, CH_{3Het}), 2.53 (m, 1H, -CHCH₂CH₂CH₂N-), 2.1-1.8 (m, 3H, 2H from -CHCH₂CH₂CH₂N-+ 1H from - CHCH₂CH₂CH₂N-) ppm.
MS-ESI (+): [M + Na]⁺ = 580.1

### Example 14: Synthesis of N-(1-(3-fluoro-2'-methoxy-[1,1'-biphenyl]-4-yl)-2-oxopiperidin-3-yl)-5-phenylthiophene-2-sulfonamide (IUCT-0463.06)

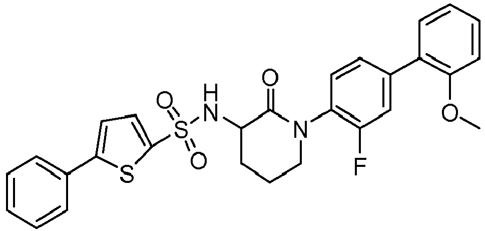

Following a procedure analogous to that described in Example 6, the title compound was obtained as a white solid in 48% yield.
¹H-NMR (400MHz, CDCl₃), δ: 8.5 (br, 1H, CHNHSO₂), 7.9 (m, 1H, 1H_{Ar}), 7.8-7.3 (m, 13H_{Ar}), 3.76 (s, 3H, OCH₃), 3.59 (br, 3H, CHNHSO₂ + -CHCH₂CH₂CH₂N-), 2.76 (s, 3H, CH_{3Het}), 2.53 (m, 1H, -CHCH₂CH₂CH₂N-), 2.1-1.8 (m, 3H, 2H from -CHCH₂CH₂CH₂N-+ 1H from - CHCH₂CH₂CH₂N-) ppm.
MS-ESI (+): [M + Na]⁺ = 559.2

### Example 15: Synthesis of 4,5-Dichloro-N-(cyanomethyl)-N-(1-(3-fluoro-2'-methoxy-[1,1'-biphenyl]-4-yl)-2-oxopiperidin-3-yl)thiophene-2-sulfonamide (IUCT-0465)

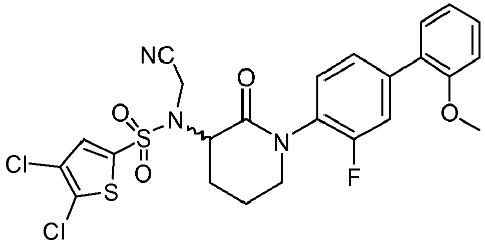

Under inert atmosphere, to a stirred solution of NaH (6.8 mg, 0.13 mmol) in 0.25 ml anhydrous DMF at 0°C, a solution of compound of Example 6 (0.030 g, 0.06 mmol) in 0.25 ml anhydrous DMF was added. After 1.5 h at 0°C, R⁵-Y (12 µl, 0.17 mmol) was added to the reaction mixture. This mixture was stirred at 0°C and the at room temperature until consumption of starting materials, and then solvent was completely removed. The crude was chromatographically purified over Al₂O₃ using Hexane/AcOEt as the eluant, yielding 6.6 mg (20 %) of the desired product.
MS-ESI (+): [M + Na]⁺ = 590.1

### Example 16: Synthesis of Ethyl N-(benzo[d][1,3]dioxol-5-ylsulfonyl)-N-(1-(3-fluoro-2'-methoxy-[1,1'-biphenyl]-4-yl)-2-oxopiperidin-3-yl)glycinate (IUCT-0466)

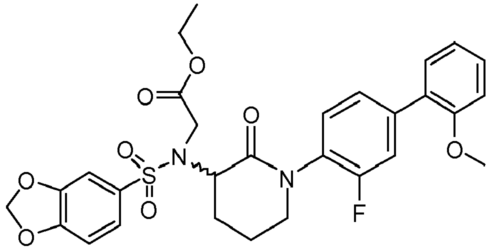

Following a procedure analogous to that described in Example 15, the title compound was obtained as a white solid in 44% yield.
¹H-NMR (400MHz, CDCl₃), δ: 7.47 (m, 1H_{Ar}), 7.40-7.15 (m, 6H_{Ar}), 6.96 (m, 2H, 2xH_{Ar}), 6.75 (d, ³*J*=8 Hz, 2H_{Ar}), 5.96 (s, 2H, OCH₂O), 4.5 (m, 1H, -NCH₂COO-), 4.13 (q, 2H, CO₂CH₂CH₃), 4.05 (d, ²*J*=20 Hz, 1H, -NCH₂COO-), 3.74 (s, 3H, OCH₃), 3.60 (d, ²*J*=20 Hz, 1H, -NCH₂COO-), 3.59 (br, 3H, CHNHSO₂ + -CHCH₂CH₂CH₂N-), 2.53 (m, 1H, -CHCH₂CH₂CH₂N-), 2.1-1.8 (m, 3H, 2H from -CHCH₂CH₂CH₂N-+ 1H from -CHCH₂CH₂CH₂N-), 1.25 (t, 3H, CO₂CH₂CH₃) ppm.
MS-ESI (+): [M + Na]⁺ = 607.2

### Example 17: Synthesis of N-(1-(3-fluoro-2'-methoxy-[1,1'-biphenyl]-4-yl)-2-oxopiperidin-3-yl)-N-(2-morpholino-2-oxoethyl)benzo[d][1.3]dioxole-5-sulfonamide (IUCT-0467)

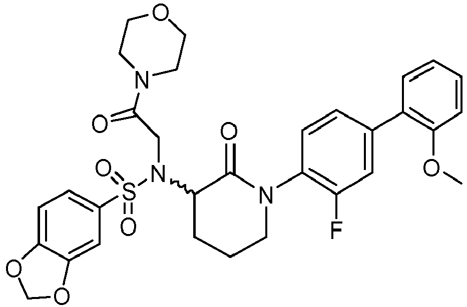

Following a procedure analogous to that described in Example 15, the title compound was obtained as a white solid in 25 % yield.
MS-ESI (+): [M + H]⁺ = 626.2, [M + Na]⁺ = 648.3

### Example 18: Synthesis of 2-((4,5-dichloro-N-(1-(3-fluoro-2'-methoxy-[1,1'-biphenyl]-4-yl)-2-oxopiperidin-3-yl)thiophene)-2-sulfonamido)acetamide (IUCT-0468)

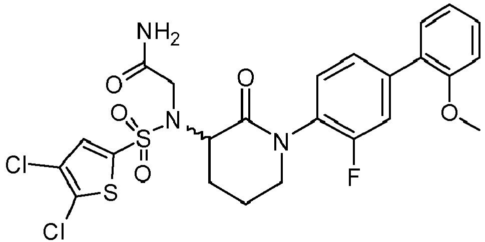

Following a procedure analogous to that described in Example 15, the title compound was obtained as a white solid in 11 % yield.
MS-ESI (+): [M + Na]⁺ = 608.1

### Example 19: Synthesis of Ethyl N-(1-(3-fluoro-2'-methoxy-[1,1'-biphenyl]-4-yl)-2-oxopiperidin-3-yl)-N-((5-(pyridin-2-yl)thiophen-2-yl)sulfonyl)glycinate (IUCT-0469)

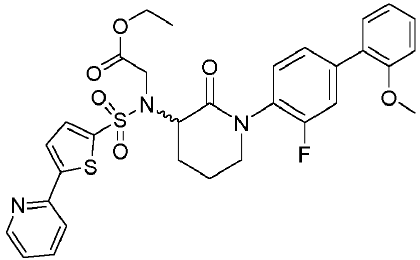

Following a procedure analogous to that described in Example 15, the title compound was obtained as a white solid in 63 % yield.
MS-ESI (+): [M + H]⁺ = 624.2, [M + Na]⁺ = 646.2

### Example 20: Compounds of formula (I) do not disrupt the CNA-CF-PRIEIT interaction, since they do not bind to PxIxIT

Compounds were analyzed for their potential to disrupt the interaction between the human catalytic domain of the calcineurin Aα subunit (CNAα) and the NFATc2-derived PRIEIT peptide. For this purpose we used Flourescence Polarization (FP) technique described in Mulero et al, 2009 (J. Biol. Chem.284:9394-9401). Briefly, to identify disruptors of the CNA-PRIEIT interaction, 5 µM of human CNAα (amino acids 2-347) is preincubated with 50 µM of each hit compound in OptiPlate black 384-well flat-bottom plates (PerkinElmer Life Sciences) for 15 min at room temperature. Then, carboxyfluorescein (CF)-tagged NFATc2-derived PRIEIT peptide (CF-PRIEIT, 30 nM) is added to each well and incubated for 15 more min at room temperature. Each compound has been analyzed once with triplicates. FP measurements were performed using a Victor™ X5 2030 Multilabel Reader (PerkinElmer Life Sciences) with excitation and emission wavelengths of 485 nm and 535 nm, respectively. The fluorescent polarization of the free CF-peptide was considered 0% binding and the binding of CNA to the CF-PRIEIT peptide as 100% binding. Accordingly, the capacity of the compound to disrupt CNA-CF-PRIEIT interaction was measured. None of them displace the CF-PRIEIT peptide from the CNA-PRIEIT interaction (Table 3). Interaction disruption values are less than 30%.

**Table 3: Compounds do not behave as disruptors of the CnA- CF-PRIEIT interaction by using a fluorescence polarization assay. Data is given as mean ± standard deviation (SD).**

| **Code** | **Interaction Disruption (%)** | **+/- SD (%)** |
|---|---|---|
| IUCT-0460 | 6,53 | 12,17 |
| IUCT-0461 | 11,09 | 4,83 |
| IUCT-0462 | 25,71 | 6,57 |
| IUCT-0463 | 15,75 | 9,57 |
| IUCT-0464 | 25,83 | 12,16 |
| IUCT-0465 | 13,79 | 6,37 |
| IUCT-0466 | 16,69 | 9,96 |
| IUCT-0469 | 29,26 | 9,39 |

### Example 21: Compounds of formula (I) do not disrupt the CNA-CF-PRIEIT interaction.

INK compounds were analyzed for their ability to disrupt CN-PRIEIT interaction using a fluorescence polarization assay. This biochemical assay requires recombinant human CNAD (amino acids 2-347) fused to glutathione S-transferase (GST-CN), which was expressed and purified from bacteria, and a NFATc2-derived PRIEIT peptide labeled with carboxyfluorescein (CF), CF-PRIEIT.
Competition binding assays of INK compounds on the interaction of CN with the CF-NFATc2-derived PRIEIT peptide were performed by fluorescence polarization. Briefly, 6 µM of CNA was incubated in the absence (positive control of interaction) or in the presence of 50 µM or 10 µM of competitor compound prior to addition of 30 nM of CF-SPRIEIT peptide. VIVIT peptide was included as positive control of interaction disruption ability. Data is represented as mean percentage of CF-SPRIEIT bound to CNA (± SEM) of 2 experiments performed in triplicates. Signal in the absence of competing peptide was considered as 100% of CF-SPRIEIT bound to CNA.

**Table 4: Functional effect of pair related structure compounds on NFATc activation**

| Relative interaction (%) | Sample at 50 µM | | | Mean | SD (%) | Sample at 10 µM | | | Mean | SD (%) |
|---|---|---|---|---|---|---|---|---|---|---|
| Control | 108,5 | 97,3 | 100 | 101,9 | 5,8 | 100,6 | 93,5 | 93,6 | 95,9 | 4,1 |
| VIVIT peptide | 20,4 | 16,0 | 17,6 | 18,0 | 2,2 | 36,4 | 31,2 | 31,3 | 33,0 | 3,0 |
| IUCT-0464 | 79,0 | 87,7 | 82,9 | 83,2 | 4,4 | 96,8 | 92,3 | 92,4 | 93,8 | 2,6 |

### Example 22: Compounds of formula (I) do not bind to PxIxIT nor LxVP binding sites

Compounds of formula (I) were confronted by docking to the crystallized structure of calcineurin subunit A. The crystal structure of calcineurina was extracted from Protein Data Bank: 3LL8 (https://www.rcsb.org/structure/3ll8). The Blind Docking Kin software from Mind the Byte's SaaS Platform has been used for this purpose (https://www.mindthebyte.com/products/saas-platform/).
Accordingly, several possible interactions of each molecule in front of several cavities of the protein were explored (see Table 5).
Surprisingly, compounds of formula (I) exhibit very low binding energies at PxIxIT pocket, and moderate binding energies at LxVP site. However, even more surprisingly, binding energies were high and therefore very effective at a different binding site, renamed as IUCT's pocket.

**Table 5**

| **Code** | **Binding energy (Kcal/mol) at different sites** | | |
|---|---|---|---|
| | **PxIxIT** | **LxVP** | **IUCT pocket** |
| IUCT-0464 | -2,7 | -7,7 | -11 |
| IUCT-0463 | -1,4 | -6,2 | -10,1 |
| IUCT-0462 | -2,5 | -6,7 | -9,9 |
| IUCT-0463.06 | -0,6 | -6,1 | -9,9 |
| IUCT-0463.04 | -3,5 | -5,5 | -9,8 |
| IUCT-0463.02 | -3,4 | -7,1 | -9,5 |
| IUCT-0469 | n.b. | -3,8 | -9,4 |
| IUCT-0463.03 | -3,5 | -5,3 | -9,3 |
| IUCT-0461 | -0,2 | -5,4 | -9 |
| IUCT-0465 | -1,4 | -6 | -9 |
| IUCT-0466 | n.b. | -5,9 | -9 |
| IUCT-0463.01 | -2,5 | -5,5 | -8,6 |
| IUCT-0463.05 | n.b. | -5,7 | -8,4 |
| IUCT-0460 | -2,5 | -5,7 | -8,2 |

### Example 23: Compounds of formula (I) inhibit NFATc activation in HEK 293T cells

To determine the ffect of INK compounds on NFATc activation, HEK 293T cells were transfected with 3xNFAT-luc reporter gene and Renilla-luc plasmid as control of gene transfection. After 24 hours, to activate the cellular CN -NFATc signaling pathway, cells were stimulated with ionomycin (lo), PMA and CaCl2 for 4 h to raise intracellular concentration of calcium. As positive control of inhibition of CN activity, cells were treated with 1 µM of CsA 30 min prior lo/PMA/CaCl2 stimulation. Then, cells were treated with compounds of formula (I) at several concentrations for 2 hours and light units analyzed following manufacture's protocol of the dual-luciferase reporter assay system (Figure 1, Functional effect of pair related structure compounds on NFATc activation).

Results from Figure 1 show that IUCT-0464 strongly inhibits NFATc activation in HEK 293T cells. This inhibition takes place at least at 100, 50 and 20 µM of the compound concentration.

### Example 24: Compounds of formula (I) do not inhibit calcineurin phosphatase activity towards the pNPP substrate

CN dephosphorylation activity is commonly evaluated towards the small molecule pNPP, to determine the accessibility of small molecules to the active site of CN enzyme, or towards the RII peptide, from the regulatory RII subunit of cAMP-dependent protein kinase, as a big substrate that binds to CNA prior dephosphorylation by the CN active site.
To determine the functional effect of compounds of formula (I) on CN phosphatase activity towards the pNPP substrate, a biochemical assay was performed. The assay evaluates the ability of recombinant human CNA (amino acids 2-347) fused to glutathione S-transferase (GST-CN) to dephosphorylate the pNPP substrate. First, GST-CN was expressed at high amounts in bacteria, purified and then CN activity analyzed, in the absence of any compound, towards the pNPP substrate. Then, 50 µM of each compound was analyzed to determine their inhibitory potential towards the phosphatase activity of CN (Figure 2).

### Example 25: Compounds of formula (I) do not inhibit calcineurin phosphatase activity towards the RII substrate.

Compounds that inhibit NFATc activity were also analyzed for their ability to inhibit CN full length protein towards the RII substrate by using the CN phosphatase assay kit (Enzo Life Sciences) and following manufacturer's instructions. °
Compounds of formula (I) do not seem to affect phosphatase activity of CNA towards the RII substrate (Figure 3).

In conclusion, compounds of formula (I) do not disrupt CN-NFATc interaction. Compounds of formula (I) do not affect the phosphatase activity of CNA towards pNPP or RII substrates. Compounds of formula (I) show a strong inhibitory effect of NFATc activation in human HEK 293T cells at several concentrations.

However, compounds of formula (I) compete with NFATc binding to CN by a site different from the previously described PxIxIT- and LxVP- binding motifs on CNA.

## Claims

1. A compound having formula (I) 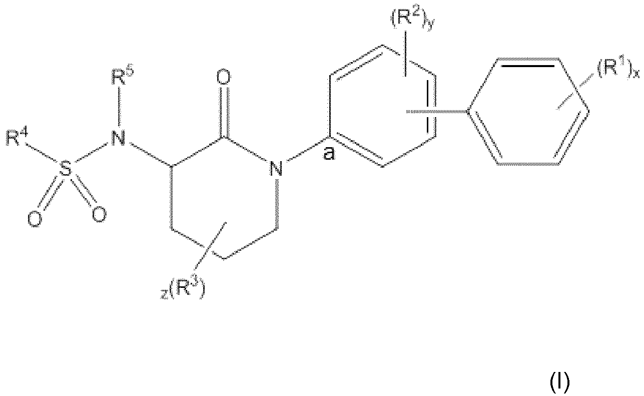 and the salts and stereoisomers thereof, wherein
**R¹** is hydrogen; OH; NH₂; SH; F; Cl; Br; I; methyl; an optionally substituted alkyl chain provided that said optionally substituted alkyl chain is not CH₂NEt₂, optionally substituted CH₂-pirrolidine, optionally substituted CH₂-piperidine or CH₂NMeCH₂CH₂OH; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NHCONR⁶R⁷; NHCOR⁶; NR⁶CO₂R⁷; NHCO₂R⁶; NHC(S)OR⁶; NO₂; N₃; SR⁸; SO₃H; SO₂R⁸; SO₂NR⁶R⁹; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
wherein x is one, two, three, four or five, independently of y, z;
wherein the phenyl ring bearing substituent **R¹** is in an orto, meta or para position regarding the position "a" of the phenyl ring bearing substituent **R²;**
**R²** is hydrogen; OH; NH₂; SH; F; Cl; Br; I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
wherein **y** is one, two, three, four or five, independently of x, z;
**R³** is hydrogen; OH; NH₂; SH; F; Cl; Br; I; methyl; an optionally substituted alkyl chain; an optionally substituted alkenyl chain; an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
wherein **z** is one, two, three, four or five, independently of x, y;
**R⁴** is hydrogen; OH; NH₂; methyl; an optionally substituted alkyl chain; an optionally substituted alkenyl chain; an optionally substituted alkynyl chain; an optionally substituted cycloalkyl linked to S through an optionally substituted alkyl chain; an optionally substituted cycloalkyl linked to S through an optionally substituted alkenyl chain; an optionally substituted cycloalkyl linked to S through an optionally substituted alkynyl chain; an optionally substituted cycloalkenyl; an optionally substituted cycloalkenyl linked to S through an optionally substituted alkyl chain; an optionally substituted cycloalkenyl linked to S through an optionally substituted alkenyl chain; an optionally substituted cycloalkenyl linked to S through an optionally substituted alkynyl chain; an optionally substituted cycloalkynyl chain; an optionally substituted cycloalkynyl linked to S through an optionally substituted alkyl chain; an optionally substituted cycloalkynyl linked to S through an optionally substituted alkenyl chain; an optionally substituted cycloalkynyl linked to S through an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
**R⁵** is hydrogen; OH; NH₂; methyl; an optionally substituted alkyl chain; an optionally substituted alkenyl chain; an optionally substituted alkynyl chain; an optionally substituted cycloalkyl linked to S through an optionally substituted alkyl chain; an optionally substituted cycloalkyl linked to S through an optionally substituted alkenyl chain; an optionally substituted cycloalkyl linked to S through an optionally substituted alkynyl chain; an optionally substituted cycloalkenyl; an optionally substituted cycloalkenyl linked to S through an optionally substituted alkyl chain; an optionally substituted cycloalkenyl linked to S through an optionally substituted alkenyl chain; an optionally substituted cycloalkenyl linked to S through an optionally substituted alkynyl chain; an optionally substituted cycloalkynyl chain; an optionally substituted cycloalkynyl linked to S through an optionally substituted alkyl chain; an optionally substituted cycloalkynyl linked to S through an optionally substituted alkenyl chain; an optionally substituted cycloalkynyl linked to S through an optionally substituted alkynyl chain; trihaloalkyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; NO₂; N₃; NHCONR⁶R⁷; NHCOR⁶; NR⁶CO₂R⁷; NHCO₂R⁶; NHC(S)OR⁶; NO₂; SO₃H; SO₂R⁶; SO₂NR⁶R⁷; aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain;
**R⁶** and **R⁷** are selected, independently of each other, from hydrogen; methyl; an optionally substituted alkyl chain; an optionally substituted alkenyl chain; an optionally substituted alkynyl chain; an optionally substituted heterocycle; and an optionally substituted aryl, preferably phenyl or naphthyl;
**R⁸** is selected from hydrogen; an optionally substituted alkyl chain except methyl; an optionally substituted alkenyl chain; an optionally substituted alkynyl chain; an optionally substituted heterocycle; and an optionally substituted aryl, preferably phenyl or naphthyl;
**R⁹** is selected, independently of **R⁶**, from an optionally substituted alkyl chain; an optionally substituted alkenyl chain; an optionally substituted alkynyl chain; an optionally substituted heterocycle; and an optionally substituted aryl, preferably phenyl or naphthyl;
each **aryl** as a group or part of a group is phenyl, naphthalenyl, anthracenyl, phenantrenyl, biphenyl, optionally fused to Het; each optionally substituted with one, two, three or four substituents selected from OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain;
each **Het** as a group or part of a group is a monocyclic ring with 5, 6 or 7 ring atoms optionally fused to an aryl or a bicyclic ring structure comprising a 5, 6 or 7 membered ring linked to a 4, 5, or 6 membered ring; each of the rings being saturated, partially unsaturated, or completely unsaturated; at least one of the rings containing 1 to 5 heteroatoms each independently selected from nitrogen, oxygen and sulphur; and any one of the rings being optionally substituted with one, two, three or four substituents each independently selected from the group consisting of OH; F; Cl; Br; I; methyl; an optionally substituted alkyl chain; an optionally substituted alkenyl chain; an optionally substituted alkynyl chain; an optionally substituted cycloalkyl; an optionally substituted cycloalkenyl; an optionally substituted cycloalkynyl; OR⁶; NR⁶R⁷; CN; COR⁶; CONR⁶R⁷; CO₂R⁶; C(S)OR⁶; OCONR⁶R⁷; OCOR⁶; OCO₂R⁶; OC(S)OR⁶; N₃; NO₂; NHCONR⁶R⁷; NHCOR⁶; NR⁶CO₂R⁷; NHCO₂R⁶; NHC(S)OR⁶; NO₂; SR⁶; SO₃H; SO₂R⁶; SO₂NR⁶R⁷; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain;

2. The compound according to claim 1, wherein the phenyl ring bearing substituent **R¹** is in para position regarding the position "a" of the phenyl ring bearing substituent **R².**

3. The compound according to claim 1 or 2, wherein
**R³** is hydrogen.

4. The compound according to any of claims 1 to 3, wherein
**R²** is hydrogen; OH; NH₂; SH; F; Cl; Br; I; or methyl.

5. The compound according to any of claims 1 to 4, wherein
**R⁴** is aryl; Het; an optionally substituted aryl linked by an optionally substituted alkyl, alkenyl, alkynyl chain, aryl or Het; or an optionally substituted heterocycle linked by an optionally substituted alkyl, alkenyl or alkynyl chain.

6. The compound according to any of claims 1 to 5, wherein
**R¹** is hydrogen; F; Cl; Br; I; methyl; an optionally substituted alkyl chain; aryl; or Het; provided that said optionally substituted alkyl chain is not CH₂NEt₂, optionally substituted CH₂-pirrolidine, optionally substituted CH₂-piperidine or CH₂NMeCH₂CH₂OH; OR⁶;
**R²** is F; Cl; Br; I;
**R⁵** is hydrogen; methyl; an optionally substituted alkyl chain; an optionally substituted cycloalkyl linked to S through an optionally substituted alkyl chain; aryl; Het; an optionally substituted aryl linked by an optionally substituted alkyl, alkenyl, alkynyl chain, aryl or Het; and an optionally substituted heterocycle linked by an optionally substituted alkyl, alkenyl or alkynyl chain.

7. The compound according to any of claims 1 to 6, wherein
**R¹** is OR⁶.

8. The compound according to any of claims 1 to 7, wherein
**R²** is F.

9. The compound according to any of claims 1 to 8, selected from:
- 4,5-Dichloro-*N*-(1-(3-fluoro-2'-methoxy-[1,1'-biphenyl]-4-yl)-2-oxopiperidin-3-yl)thiophene-2-sulfonamide;
- *N*-(1-(3-fluoro-2'-methoxy-[1,1'-biphenyl]-4-yl)-2-oxopiperidin-3-yl)-3,5-dimethylisoxazole-4-sulfonamide;
- *N*-(1-(3-fluoro-2'-methoxy-[1,1'-biphenyl]-4-yl)-2-oxopiperidin-3-yl)benzo[d][1,3]dioxole-5-sulfonamide;
- *N*-(1-(3-fluoro-2'-methoxy-[1,1'-biphenyl]-4-yl)-2-oxopiperidin-3-yl)-5-(pyridin-2-yl)thiophene-2-sulfonamide;
- *N*-(1-(3-fluoro-2'-methoxy-[1,1'-biphenyl]-4-yl)-2-oxopiperidin-3-yl)benzofuran-2-sulfonamide;
- 4,5-Dichloro-*N*-(cyanomethyl)-*N*-(1-(3-fluoro-2'-methoxy-[1,1'-biphenyl]-4-yl)-2-oxopiperidin-3-yl)thiophene-2-sulfonamide;
- Ethyl *N*-(benzo[d][1,3]dioxol-5-ylsulfonyl)-*N*-(1-(3-fluoro-2'-methoxy-[1,1'-biphenyl]-4-yl)-2-oxopiperidin-3-yl)glycinate;
- *N*-(1-(3-fluoro-2'-methoxy-[1,1'-biphenyl]-4-yl)-2-oxopiperidin-3-yl)-*N*-(2-morpholino-2-oxoethyl)benzo[d][1,3]dioxole-5-sulfonamide;
- 2-((4,5-dichloro-*N*-(1-(3-fluoro-2'-methoxy-[1,1'-biphenyl]-4-yl)-2-oxopiperidin-3-yl)thiophene)-2-sulfonamido)acetamide;
- Ethyl *N*-(1-(3-fluoro-2'-methoxy-[1,1'-biphenyl]-4-yl)-2-oxopiperidin-3-yl)-*N*-((5-(pyridin-2-yl)thiophen-2-yl)sulfonyl)glycinate;
- *N*-(1-(3-fluoro-2'-methoxy-[1,1'-biphenyl]-4-yl)-2-oxopiperidin-3-yl)-5-(2-(methylthio) pyrimidin-4-yl)thiophene-2-sulfonamide;
- *N*-(1-(3-fluoro-2'-methoxy-[1,1'-biphenyl]-4-yl)-2-oxopiperidin-3-yl)benzo[b]thiophene-2-sulfonamide;
- *N*-(1-(3-fluoro-2'-methoxy-[1,1'-biphenyl]-4-yl)-2-oxopiperidin-3-yl)-5-(isoxazol-5-yl)thiophene-2-sulfonamide;
- *N*-(1-(3-fluoro-2'-methoxy-[1,1'-biphenyl]-4-yl)-2-oxopiperidin-3-yl)-5-(oxazol-5-yl)thiophene-2-sulfonamide;
- *N*-(1-(3-fluoro-2'-methoxy-[1,1'-biphenyl]-4-yl)-2-oxopiperidin-3-yl)-5-(2-methylthiazol-4-yl)thiophene-2-sulfonamide; and
- *N*-(1-(3-fluoro-2'-methoxy-[1,1'-biphenyl]-4-yl)-2-oxopiperidin-3-yl)-5-phenylthiophene-2-sulfonamide.

10. The compound according to claim 9 selected from:
- *N*-(1-(3-fluoro-2'-methoxy-[1,1'-biphenyl]-4-yl)-2-oxopiperidin-3-yl)-5-(pyridin-2-yl)thiophene-2-sulfonamide; and
- *N*-(1-(3-fluoro-2'-methoxy-[1,1'-biphenyl]-4-yl)-2-oxopiperidin-3-yl)benzofuran-2-sulfonamide;

11. Use of a compound according to any of claims 1 to 10 as a calcineurin inhibitor.

12. Use of a compound according to any of claims 1 to 10, as an inhibitor of the calciurenin-NFAT signaling pathway.

13. Use of a compound according to any of claims 1 to 10 as an immunosuppresive drug.

14. The compound according to any of claims 1 to 10 for use in the prevention or treatment of an autoimmune disease or organ transplant rejection.

15. The compound for use according to claim 14, wherein said autoimmune disease is selected from arthritis, anemia, psoriasis, dermatitis, urticaria, sclerosis, multiple sclerosis, inflammatory bowel disease, Crohn's disease, colitis, endocarditis, endometriosis, episcleritis, respiratory distress syndrome, meningitis, iritis, choroiditis, spondylitis, sudden hering loss, rhinitis, encephalitis, uveitis, autoimmune mocarditis, leukocyte adhesion deficiency, lupus, systemic lupus erythemotosus, diabetis mellitus, tuberculosis, sarcoidosis, granulomatosis, vasculitides, autoimmune aplastic anemia, autoimmune neutropenia, CNS inflammatory disorders, Bechet's or Behcet's disease, pemphigoid, pemphigus, Reiter's disease, nephritis, IgM polyneuropathies, thrombocytopenia, hypothyroidism, hypoparathyroidism, thyroiditis, Grave's disease, polyglandular syndromes, paraneoplastic syndromes, encephalomyelitis, myasthenia gravis, autoimmune hepatitis, Guillain-Barre syndrome, Berger's disease, dermatosis, Celiac disease, amylotrophic lateral sclerosis, coronary artery disease, autoimmune hearing loss, polychondritis, paraneoplasic syndrome, channelopathies, epilepsy, migraine, autism, fibromyalgia, multiple endocrine failure, presenile dementia, Chagas' disease, rheumatic fever, recurrent abortion, erythema multiforme, alveolitis, leprosy, malaria, leishmaniasis, dengue, endocarditis, fibrosis, cyclitis, human immunodeficiency virus (HIV) infection, Alzheimer's disease, Epstein-Barr virus infection, keratoconjuctivitis, bronchitis, chronic obstructive airway disease, idiophatic facial paralysis, chronic fatigue syndrome, febris rheumatica, hypogonadism, pancreatitis, vitiligo, acquire immune deficiency syndrome, multiple organ injury syndrome, autoimmune atrophic gastritis, rheumatic diseases, myocarditis, nephrotic syndrome, sinusitis, eosinophilia, hyperalgesia, meningitis, diabetic disorders, valvulitis.
